# EUROPEAN PATENT APPLICATION

(11) **EP 3 835 421 A1**
(43) Date of publication of application: **16.06.2021**
(21) Application number: 19383092.4
(22) Date of filing: 11.12.2019
(51) Int. Cl.: C12N 15/09, C12N 15/63, A01K 67/027

(54) **VECTORS FOR TISSUE SPECIFIC TRANSCRIPTOMICS**

(71) Applicant: Consejo Superior de Investigaciones Científicas (CSIC), 28006 Madrid (ES)
(72) Inventor: MARTÍNEZ MORALES, Juan Ramón, 41013 SEVILLA (Sevilla) (ES); CORBACHO SÁNCHEZ, Jorge, 41013 SEVILLA (Sevilla) (ES)
(74) Representative: Pons Ariño, Angel

(57) **Abstract**

The present invention relates to nucleic acid vectors, methods, uses and kits thereof for the generation of transgenic cells or non-human organism which are suitable for transcriptomic analysis. This disclosure provides transposon-based vectors which contain genetic elements which allow a genetic cassette to be incorporated into the genome of a host cell or a non-human organism. The vectors, methods, uses and kits disclosed herein allow the systematic generation of collection of lines of cells or animals suitable for dynamic and tissue-specific transcriptomic interrogation in different developmental stages or in different physiological situations. The resulting collection of lines will constitute a unique transcriptomics resource for a broad community working in developmental genetics, organ physiology or disease models.

## Description

The invention relates to a novel vector useful in the field of transformation, methods, uses and kits thereof. More particularly, the present invention relates to a modified transposon-based vector that allows the introduction of genetic elements into the genome of cells or non-human organism which facilitate transcriptomics analysis.

### BACKGROUND ART

An essential step in understanding the complexity of biological systems is understanding the gene expression dynamics of individual cells, or groups of cells (like tissues or organs), i.e., which genes are activated or repress in such cells at a given point in time, both at a basal level and in response to stimuli. The generation of transgenic organisms is of major importance in several fields of biotechnology, research, therapeutic research and medicine development. Transgenic organisms have been pivotal in discovering the function of genes and proteins, as well as providing insight into the intricated relationship between genes, mRNAs and proteins, at structural, cellular, multicellular, tissue, organ and animal levels. The transcriptional status of individual cells, achieved by the unique combinatorial activation and repression of specific genes, is an intrinsic property of each cell type and tissue type. Gene expression dynamics and specificity determine the fate, shape, mechanical properties, metabolic status, physiology, pathology, and survival of each cell. Thus, addressing the question of how individual transcriptomes vary through time and among cell types is of paramount importance in most life science disciplines.

Next Generation Sequencing has revolutionised the study of transcriptomics, the study of all RNAs expressed by an organism, by the development of RNA sequencing (RNA-seq) approach, which enables a global analysis of the transcriptome of cells, tissues and organisms. RNA-seq brought several advantages regarding previous methods, namely high reproducibility, unbiased detection of transcripts, single nucleotide resolution and quantitative estimation of transcript levels over a large dynamic range of expression. Despite its advantages, RNA-seq has been seriously limited by the acquisition of RNA samples from heterogenous cell populations, making the study of transcriptome of specific cell populations a challenge. This is particularly the case when the cell population targeted represents a small fraction of the tissue or when the level of cell heterogeneity is high in the organ of study (i.e. the brain). In this situation, the expression profiling of a given cell population may be mask by the transcriptional "noise" associated to its neighbours. The most commonly used strategy to bypass this problem is to extract RNA from a homogeneous cell suspension obtained by fluorescence-activated cell sorting (FACS). Unfortunately, cell enrichment by FACS entails long and severe dissociation procedures that may distort gene expression, among other things by inducing a cellular response to stress.

To overcome such limitations, a technique to selective labelled translating RNAs in specific cells or groups of cells was developed: Translating Ribosome Affinity Purification (TRAP). TRAP is based on the transformation of a cell or animal with a fusion gene that jointly expresses a ribosome target gene labelled with a marker gene in a specific cell/tissue/organ. The translated fusion protein will get incorporated into translating ribosomes and therefore such ribosomes will also be labelled. This allows to easily isolate the specifically labelled ribosomes from whole organism's extracts by immunoprecipitation, also obtaining the mRNA attached to them, which facilitates the generation of specific transcriptomes of cells/tissues/organs at different developmental stages. The main drawback of the TRAP technique is the difficulty, and at times the impossibility, of having the fusion gene being expressed specifically in certain cells/tissues/organs since nucleic acid elements that specifically promote expression in such targets (like promoters and upstream activating sequences) may be unknown or lack an adequate level of expression for TRAP. In addition, each different target of study requires the generation of transgenic cell/animal, a process which can be cumbersome, difficult and expensive, limiting the application of TRAP (Tryon, R.C. et al. Genesis. 2013. 51(3):187-192).

### DESCRIPTION OF THE INVENTION

The present invention discloses novel genetic constructions, vectors, methods for its production and uses thereof, which greatly facilitate the creation of libraries of transgenic non-human organism lines which are a valuable resource for the study of transcriptomics, developmental genetics, organ physiology or disease models.

The vectors of the present invention solve the problems associated with TRAP by allowing a faster, cheaper, efficient, flexible and consistent generation of transgenic non-human organisms, wherein said organisms have specific cells, tissues or organs labelled, ideal for performing TRAP. The genetic constructions and vectors comprising these genetic constructions, comprises a nucleotide sequence containing several elements that can randomly integrate into the genome of a target host cell, which will only be active if the integration happens in the proximity of an expression promoting sequence (e.g. an enhancer). Such cells can be used to generate transgenic organisms by known means in the art. The libraries of transgenic host cell and non-human transgenic organism lines created will allow not only for TRAP studies by RNA-seq but also for developmental genetics, physiology, toxicology and biomedical studies. Thus, the genetic constructions and vectors of the present invention are useful to generate transgenic lines suitable for dynamic and tissue-specific transcriptomic interrogation. The resulting collection of lines may constitute a unique resource for a broad community working in developmental genetics, organ physiology or disease models.

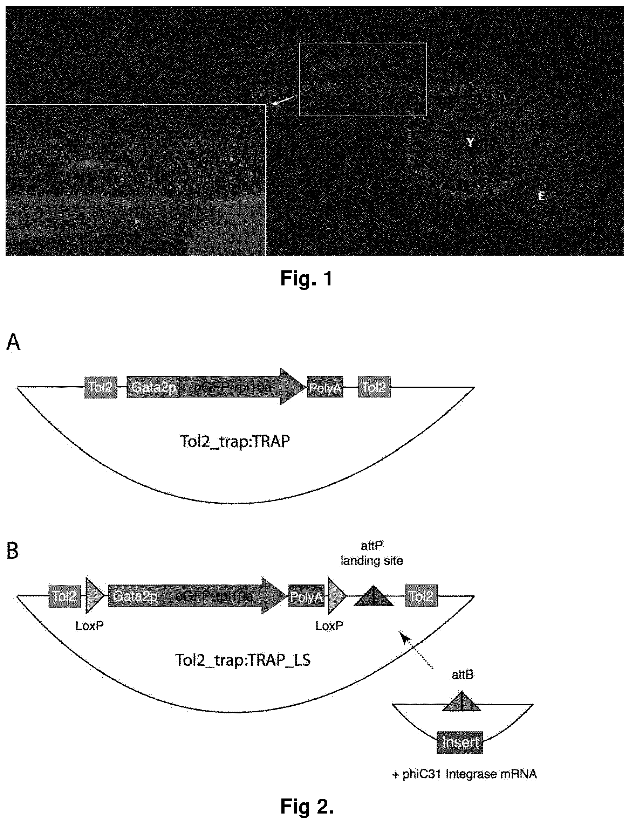

As an example of this advantages, Fig. 1 shows a line of zebrafish obtained with the vector of the invention, particularly with the vector shows in Fig. 2A where the tissue lateral line is labelled specifically. It is important to note that is the first time that this tissue has been labelled in such a way, which will allow for studies on the differentiation program and migratory properties of this organ, a reference model for collective cell migration. This is a key process involved in the morphogenesis of diverse organ systems and it has been also identified in wound healing (Scarpa, E., Mayor R. J. Cell. Biol. 2016; 212:143-155). Moreover, collective cell migration is a capacity observed in cancer invasion and metastasis (Lintz, M. et al. J. Biomech. Eng. 2017;139:021005). Therefore, this line can be extremely useful to gain insight into these processes and study mechanisms as cell-cell cohesion, intercellular communication, response to chemotactics or the role of tissue-derived factors that guide the collective migration.

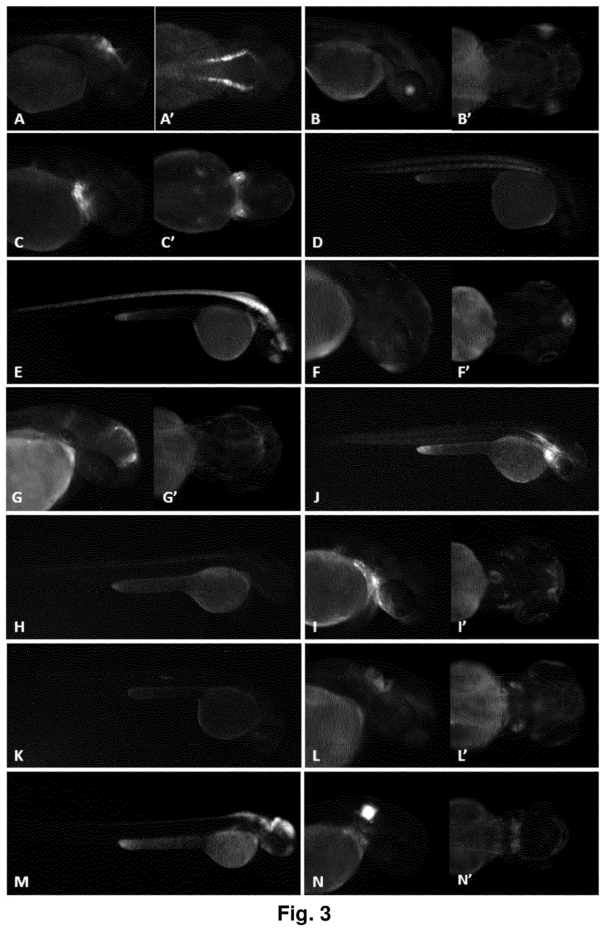

Another example of the benefices of the genetic construction and the vector disclosed herein are the labelling of the entire nervous system as it is shown in Fig. 3E, which includes several neuromeres, but which are globally labelled allowing the analysis of the whole organ. This line makes it possible to perform analysis to investigate how the whole organ is developed, involving the formation of neuromeres, which can be very useful for neurogenesis studies as induction of neural progenitors, specification and differentiation to neurons.

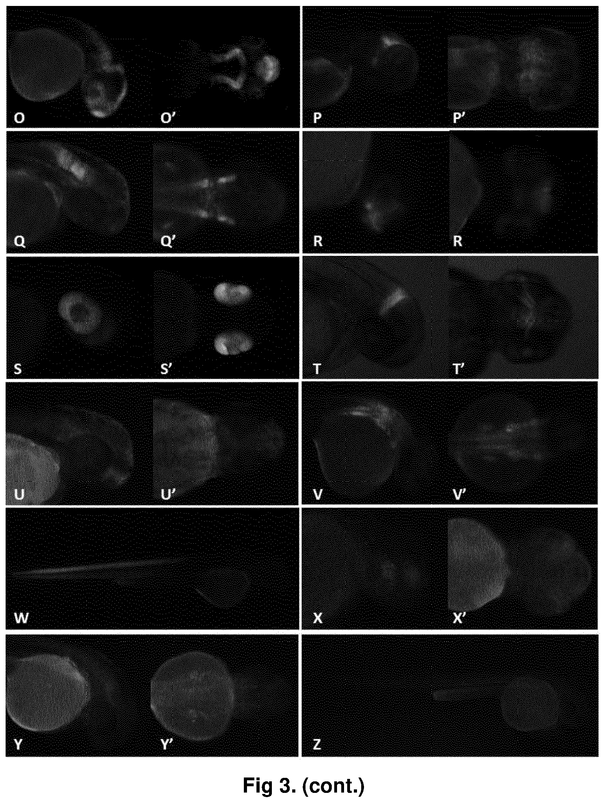

Other example of the utility and versatility of the genetic construction is the specific labelling of the optic cup Fig. 3S, S'. The anatomy and physiology of the eye is very conserved in most classes of vertebrates. The zebrafish eye has become a great model for studies on eye behaviour and morphogenesis as well as investigation on human eye diseases basis (Richardson, R. et al. Eye. 2017;31:68-86).

Thus, the invention also relates to a non-human cell and non-human organism which comprises the above-mentioned genetic construction or vector. Moreover, the invention relates to the use of said non-human cell or non-human organism for identification and/or isolation of genes and for the creation of transgenic organisms to study gene function at various developmental stages, including the adult, as well as for the creation of animal models of animal and/or human diseases useful for drug target validation.

In a first aspect, the present invention relates to a genetic cassette, hereinafter genetic cassette of the invention, comprising
a. a pair of transposase binding sites delimiting said genetic cassette, and
b. a fusion gene comprising
   i. a reporter gene, and
   ii. a ribosomal gene
wherein the fusion gene is in operational combination with a minimal promoter.

The term "genetic cassette" as used herein refers to any polynucleotide sequence having a discrete and identifiable structure, a fragment or segment of DNA containing a particular grouping of genetic elements. The genetic cassette may be inserted into the target genome. In addition, a genetic cassette can include one or more nucleic acid sequences. An example of a genetic cassette is one that contains at least one, preferably two transposase binding sites, at least one promoter element and one or more nucleic acid for expression. Additionally, the genetic cassette can also comprise a polyadenylation sequence.

In a specific embodiment, the presence of two transposase binding sites in the expression cassette of the invention means that the genetic cassette has the capacity of insertion into the genome of a host cell upon introduction into said host cell, and therefore the genetic cassette can be replicated along with the genome of said host cell.

In another specific embodiment, the nature of the polyadenylation signal is not believed to be crucial to the successful practice of the methods of the present disclosure, and any such sequence may be employed, including but not limited to the SV40 early polyadenylation signal, the SV40 late polyadenylation signal, the HSV thymidine kinase polyadenylation signal, the protamine gene polyadenylation signal, the adenovirus 5 Elb polyadenylation signal, the bovine growth hormone polyadenylation signal, the human variant growth hormone polyadenylation signal and the rabbit beta-globin polyadenylation signal.

In a preferred embodiment, the polyadenylation signal is SV40polyA or functional variants and fragments thereof. In more preferred embodiments the polyadenylation signal is from the polyomavirus simian virus 40 (SV40). In a specific embodiment, the SV40polyA comprise a nucleotide sequence having at least 90% sequence identity with the SEQ ID NO: 1, preferably a 91, 92, 93, 94, 95, 96, 97, 98, 99 or 100% identity with the SEQ ID NO: 1. In a more preferred embodiment, the SV40polyA comprise the SEQ ID NO: 1. In a more preferred embodiment the SV40polyA consisting of the SEQ ID NO: 1.

The term "identity", as used in this specification, refers to the proportion of identical amino acids or nucleotides between two compared peptides/proteins or nucleotide sequences, respectively. The methods for comparing sequences are known in the state of the art, and include, but not limited to, the programs BLASTP or BLASTN, ClustalW and FASTA. We can consider that peptides, proteins or nucleotide sequences with percent identities of at least 90% will maintain the same properties as the sequence to which they refer.

In a more preferred embodiment, the genetic cassette of the invention can be removed and inserted into a vector or plasmid as a single unit.

The term "nucleic acid" used herein refers to nucleic acid molecules including DNA (gDNA, cDNA), oligonucleotides (double or single stranded), RNAs (sense RNAs, antisense RNAs, mRNAs, tRNAs, rRNAs, small interfering RNAs (siRNAs), doublestranded RNAs (dsRNA), short hairpin RNAs (shRNAs), piwi-interacting RNAs (PiRNA), micro RNAs (miRNAs), small nucleolar RNAs -(SnoRNAs), small nuclear RNAs (SnRNAs)), ribozymes, aptamers, DNAzymes, ribonuclease-type complexes and other such molecules as herein described. For the avoidance of doubt, the term "nucleic acid" includes non-naturally occurring modified forms, as well as naturally occurring forms.

A "transposase binding sequence" or "transposase binding site" as used herein refers to the nucleotide sequences that delimit a genetic cassette where a transposase specifically binds when mediating transposition. The transposase binding site may comprise more than one site for binding transposase subunits. Several transposase binding sites are known in the art and a skilful person in the subject matter will be able to identify an ideal transposase binding site for use in the present invention.

A "transposition reaction" as used herein refers to a rearrangement reaction where a genetic cassette is mobilized from a donor nucleic acid into a target genome. Primary components in a transposition reaction are a genetic cassette and a transposase or an integrase enzyme.

The term "transposase" as used herein refers to an enzyme that is a component of a functional nucleic acid-protein complex having the ability to recognize and bind to one end of a genetic cassette or end sequences of a genetic cassette in a rearrangement reaction to promote the mobilization of the genetic cassette. The term "transposase" also refers to integrases from retrotransposons or of retroviral origin.

In a preferred embodiment, the genetic cassette of the present invention comprises a pair of transposase binding sites recognised by a transposase selected from a list consisting of: AC7, Tn5SEQ1, Tn916, Tn951, Tn1721, Tn2410, Tn1681, Tn1, Tn2, Tn3, Tn4, Tn5, Tn6, Tn9, Tn10, Tn30, Tn101, Tn903, Tn501, Tn1000 (γδ), Tn1681, Tn2901, AC/Ds transposons, Mp transposons, Spm transposons, En transposons, Dotted transposons, Mu transposons, dSpm transposons, I transposons, Mariner, Tc3, Mos1, Sleeping Beauty and Tol2. More preferably, the transposase binding sites are recognized by a transposase selected from a list consisting of: Mariner, Tc3, Sleeping Beauty and Tol2, more preferably, Sleeping Beauty and Tol2, and more preferably Tol2.

In a specific embodiment, the Sleeping Beauty transposase comprise an amino acid sequence having at least 90%, sequence identity with the SEQ ID NO: 2, preferably a 91, 92, 93, 94, 95, 96, 97, 98, 99 or 100% identity with the SEQ ID NO: 2. In a more preferred embodiment, the Sleeping Beauty transposase comprises the SEQ ID NO: 2. In a more preferred embodiment the Sleeping Beauty transposase consisting of the SEQ ID NO: 2.

In a specific embodiment, the Tol2 transposase comprise an amino acid sequence having at least 90% sequence identity with the SEQ ID NO: 3, preferably a 91, 92, 93, 94, 95, 96, 97, 98, 99 or 100% identity with the SEQ ID NO: 3. In a more preferred embodiment, the Tol2 transposase comprises the SEQ ID NO: 3. In a more preferred embodiment the Tol2 transposase consisting of the SEQ ID NO: 3.

In a preferred embodiment, the pair of transposase binding sites are recognized by the Tol2 transposase as described above. Thus, in a preferred embodiment the pair of transposase binding sites comprises the nucleotide sequence SEQ ID NO: 4 as first Tol2 transposase recognition sequence and SEQ ID NO: 5 as second Tol2 transposase recognition sequence.

The term "fusion gene" as used herein refers to the coding regions of two or more genes are connected end to end and placed under the control of the same set of regulatory sequences (including promoters, enhancers, ribosome binding sequences, terminators, etc.), i.e. a chimeric gene or a chimeric DNA. The expression product of the fusion gene is a fusion protein. In certain embodiments the fusion gene in addition comprises a polyadenylation signal, as described above. As it is mentioned previously, the fusion gene of the genetic cassette of the invention comprises a reporter gene and a ribosomal gene.

The term "reporter gene" as used herein refers to a nucleic acid encoding an identifying factor that is able to be identified based upon the reporter gene's effect, wherein the effect is used to track the inheritance of a nucleic acid of interest, to identify a cell, tissue, organ or organism that has inherited the nucleic acid of interest, and/or to measure gene expression induction or transcription. Selectable marker genes may also be considered reporter genes. Examples of reporter genes known and used in the art can elected from known database such as FPbase (https://www.fpbase.org) or similar.

In a particular embodiment, examples of reporter genes useful in the genetic cassette of the present invention include: luciferase (Luc), green fluorescent protein (GFP), enhanced green fluorescent protein (EGFP) red fluorescent protein (RFP), Cyan fluorescent protein (CFP), Yellow fluorescent protein (YFP), chloramphenicol acetyltransferase (CAT), β-galactosidase (LacZ), β-glucuronidase (Gus), and the like. In a more preferred embodiment, the marker gene is GFP, more preferably EGFP. In a more preferably embodiment, the EGFP gene comprises a nucleotide sequence having at least 90% sequence identity with the SEQ ID NO: 6, preferably a 91, 92, 93, 94, 95, 96, 97, 98, 99 or 100% identity with the SEQ ID NO: 6. In a more preferred embodiment, the EGFP gene comprises the SEQ ID NO: 6 and more preferably the EGFP gene consisting of the SEQ ID NO: 6. Additionally, the EGFP gene according to the present invention encodes for the EGFP protein comprising an amino acid sequence having at least 90% sequence identity with the SEQ ID NO: 7, preferably a 91, 92, 93, 94, 95, 96, 97, 98, 99 or 100% identity with the SEQ ID NO: 7. In a more preferred embodiment, the EGFP comprises the SEQ ID NO: 7 and more preferably the EGFP consisting of the SEQ ID NO: 7.

The term "ribosomal gene" as used herein refers to a gene that expresses a protein that binds to, or associates to, or is part of the ribosome. In some embodiments, the gene is a ribosomal protein gene. In some embodiments, the gene is a large ribosomal subunit protein gene. In some embodiments, the gene is a small ribosomal subunit protein gene. In specific embodiments, the gene is the ribosomal protein L10a gene (rpL10a). In a specific embodiment the ribosomal protein L10a gene is endogenous to the target cell. In a specific embodiment the ribosomal protein L10a is exogenous to the target cell. In a particular embodiment the ribosomal protein L10a gene belongs to a species selected from: mammals, avian, amphibian and fish. In a more preferred embodiment, the ribosomal protein L10a gene belongs to rodents or fish, more particularly belong to mouse or to zebrafish.

In a more particular embodiment, the ribosomal protein L10a gene from mouse comprises a nucleotide sequence having at least 90% sequence identity with the SEQ ID NO: 8, preferably a 91, 92, 93, 94, 95, 96, 97, 98, 99 or 100% identity with the SEQ ID NO: 8. In a more preferred embodiment the ribosomal protein L10a from mouse comprises the SEQ ID NO: 8 and more preferably consists of the SEQ ID NO: 8. In a more particular embodiment, the ribosomal protein L10a gene according to the present invention, encodes for the rpL10a protein comprising an amino acid sequence having at least 90% sequence identity with the SEQ ID NO: 9, preferably a 91, 92, 93, 94, 95, 96, 97, 98, 99 or 100% identity with the SEQ ID NO: 9. In a more preferably embodiment the ribosomal protein L10a from mouse comprises the SEQ ID NO: 9 and more preferably consists of the SEQ ID NO: 9.

In a more particular embodiment, the ribosomal protein L10a gene from zebrafish comprises a nucleotide sequence having at least 90% sequence identity with the SEQ ID NO: 10, preferably a 91, 92, 93, 94, 95, 96, 97, 98, 99 or 100% identity with the SEQ ID NO: 10. In a more preferably embodiment the ribosomal protein L10a gene from zebrafish comprises the SEQ ID NO: 10 and more preferably consists of the SEQ ID NO: 10. In a more preferred embodiment, the ribosomal protein L10a gene according to the present invention encodes the rpL10a protein which comprises an amino acid sequence having at least 90% sequence identity with the SEQ ID NO: 11, preferably a 91, 92, 93, 94, 95, 96, 97, 98, 99 or 100% identity with the SEQ ID NO: 11. In a more preferably embodiment the ribosomal protein L10a from zebrafish comprises the SEQ ID NO: 11 and more preferably consists of the SEQ ID NO: 11.

In a more preferred embodiment, the fusion gene of the genetic cassette comprising as reporter gene, the nucleotide sequence comprising the SEQ ID NO: 6 which encodes for the EGFP protein (SEQ ID NO:7), as the ribosomal gene, the ribosomal protein L10a gene from zebrafish which comprises the nucleotide sequence comprising the SEQ ID NO: 10 which encodes for the rpl10a protein (SEQ ID NO: 11). In a more preferred embodiment, the fusion gene comprises the SEQ ID NO: 12.

The term "operational combination" as used herein refers to that separate nucleic acid sequences are functionally associated such that an event at one can precipitate a response from the other. Two or more operably linked nucleic acid sequences can, in combination, comprise an independent genetic element, such as an expression cassette.

The term "promoter" as used herein refers to a polynucleotide required for transcription at significant levels of a second polynucleotide to which it is operably combined.

The term "minimal promoter" as used herein refers to promoter that is sensitive, i.e., capable of responding efficiently to different enhancers, and specific, i.e., not able to drive any pattern of expression on its own. Specifically, a minimal promoter refers to a short DNA segment which is typically inactive by itself, but can mediate strong transcription when combined with other transcription regulatory elements. A minimal promoter comprises a transcription start site and functional sequences for binding the transcription start complex (TATA-box) inside a cell, a host cell, or a host organism. Several minimal promoters are known in the art and a skilful person in the subject matter will be able to identify an ideal minimal promoter for use in the specific application.

In a preferred embodiment the minimal promoter is selected from a list consisting of: ef1a, gata1 promoter (gata1p), gata2 promoter (gata2p), keratin8 (krt8), keratin4 (krt4), hsp70, c-fos, E1b, thymidine kinase (TK), carp β-actin and Hspa1a minimal promoters. In a more preferred embodiment, the minimal promoter is the gata2 promoter, more particularly the gata2 promoter belong to *Dario rerio* (zebrafish), and more particularly, comprise a nucleotide sequence having at least 90% sequence identity to SEQ ID NO: 13, preferably a 91, 92, 93, 94, 95, 96, 97, 98, 99 or 100% identity with the SEQ ID NO: 4. In a preferred embodiment, the gata2 promoter belong to *Dario rerio* comprising the SEQ ID NO: 13 and more preferably, consisting of the SEQ ID NO: 13.

In a more preferred embodiment, the genetic cassette of the invention comprises the pair of transposase binding sites correspond to the transposase Tol2 and the fusion gene comprises the EGFP as reporter gene and the ribosomal protein L10a gene from zebrafish as ribosomal gene and the Gata2p from zebrafish as a minimal promoter. Thus, in a more preferred embodiment the genetic cassette of the invention comprises the nucleotide sequence SEQ ID NO: 14.

In a more preferred embodiment, the genetic cassette of the invention further comprising a pair of specific recombination sites, preferably a pair of Cre recombinase recognition sites wherein the first Cre recombinase recognition site is located upstream of the minimal promoter and the second Cre recombinase recognition site is located downstream of the fusion gene.

As used herein, "site specific recombination sites" means a pair of DNA segments that is recognized by a site-specific recombinase in a process that allows the excision of the DNA between the pair of DNA segments. For instance, Cre recombinase recognizes either IoxP recombination sites or lox511 recombination sites which are hetero-specific, which means that IoxP and lox511 do not recombine together. The Cre/lox system is disclosed by Odell et al., Plant Physiol, 106(2): 447-58 (1994).

The term "Cre recombinase" as used herein refers to the site-specific DNA recombinase derived from the Pi bacteriophage. Cre recombinase is a 38-kDa product of the cre (cyclization recombination) gene of bacteriophage Pi and is a site-specific DNA recombinase of the nt family (Sternberg, N. et al. J. Mol. Biol. 1986. 187:197-212). Cre recombinase recognizes a 34-bp sequence on the Pi genome called LoxP (locus of X-over Pi, LoxP site) and efficiently catalyses reciprocal conservative DNA recombination between pairs of LoxP sites. Cre recombinase-mediated recombination between two directly repeated LoxP sites results in excision of DNA between.

The term "lox site" as used herein refers to a nucleotide sequence at which the product of the Cre gene of bacteriophage P1, the Cre recombinase, can catalyse a site-specific recombination event. A variety of lox sites are known in the art, including the naturally occurring IoxP, IoxB, IoxL and IoxR, as well as a number of mutant, or variant, lox sites, such as loxP511, loxP514, loxΔ86, loxΔ117, loxC2, loxP2, loxP3 and lox P23.

In a specific embodiment, the pair of Cre recombinase recognition sites, particularly the pair of LoxP sites, comprises a nucleotide sequence having at least 90% identity to the SEQ ID NO: 15, preferably a 91, 92, 93, 94, 95, 96, 97, 98, 99 or 100% identity with the SEQ ID NO: 15. In a further preferred embodiment, the pair of LoxP sites comprises the SEQ ID NO: 15, more preferably consisting of SEQ ID NO: 15.

An alternative recombinase is a FLP recombinase. As used herein, the term "FLP recombinase" refers to a site-specific DNA recombinase derived from yeast, the 2pi plasmid of *Saccharomyces cerevisiae,* that recognizes a 34 base pair DNA sequence, termed the "FRT site" (FLP recombinase target). It is also recognized by one skilled in the art that modified forms or mutants of FLP recombinase can recognize the FRT site and its variants. Therefore, the term "FLP recombinase" and "FRT site" includes all variants and mutants carrying out their functions as described above.

As used herein, the terms "upstream" and "downstream" are used to differentiate relative positions in DNA or RNA sequences. The upstream position is a position towards the 5' end of another nucleic acid segment (e.g. a promoter, gene, restriction site, etc.) in a single-stranded DNA or RNA molecule. The downstream position is a position towards the 3' of another segment of nucleic acid in a single stranded DNA or RNA molecule.

In another preferred embodiment, the genetic cassette of the invention further comprising a nucleotide sequence for an integrase specific site.

The term "integrase specific site" or "ISS" as used herein means *attP* or *attB.* When a first ISS is *attP,* a corresponding second ISS is *attB* (or the first ISS is *attB* and the second ISS is *attP*) such that the first ISS and the second ISS can undergo site specific integration mediated by or in presence of an integrase. In a preferred embodiment the ISS is located outside the region delimited by the Cre recombinase recognition sites. Preferably, site specific integration is mediated by the action of integrases which act unidirectional. Said integrases therefore increase the likelihood of persistent integration as they cannot also mediate excision of the integrated sequence in contrast to bidirectional recombinases. Accordingly, in a preferred embodiment the ISS is a sequence recognizable by an integrase.

In another preferred embodiment the integrase specific site is *attP* and comprises the nucleotide sequence SEQ ID NO: 16.

In a more preferred embodiment, the genetic cassette of the invention comprises the pair of transposase binding sites correspond to the transposase Tol2, the fusion gene comprises the EGFP as reporter gene and the ribosomal protein L10a gene from zebrafish as ribosomal gene, the Gata2p from zebrafish as the minimal promoter, the LoxP as the Cre recombinase recognition site and an integrase specific site. Thus, in a more preferred embodiment, the genetic cassette of the invention comprises the nucleotide sequence SEQ ID NO: 17.

A further aspect of the present invention relates to a vector, hereinafter the vector of the invention, comprising the genetic cassette of the invention.

The term "vector" as used herein refers to a nucleic acid molecule capable of transporting one or more other nucleic acid sequence(s), like a genetic cassette, to which it has been linked or which was introduced into said vector. One type of vector is a "plasmid", which refers to a circular double stranded DNA into which additional DNA segments may be cloned. Another type of vector is a viral vector, wherein additional DNA segments may be cloned into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) or parts thereof, are integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" or simply "expression vectors". In general, expression vectors of utility in recombinant DNA techniques are often in the form of plasmids. In the present specification, "plasmid" and "vector" may be used interchangeably, as the plasmid is the most commonly used form of vector. However, the disclosure is intended to include other forms of expression vectors, such as viral vectors (e.g., replication defective retroviruses, adenoviruses and adeno-associated viruses), which serve equivalent functions. The vector may also contain additional sequences, such as a "polylinker" or "multiple cloning site" for subcloning of additional nucleic acid sequences. A "vector backbone" or "plasmid backbone" refers to a piece of DNA containing at least a plasmid origin of replication and a selectable marker which allows for selection of hosts cells containing the plasmid or vector. When a particular plasmid or vector is modified to contain non-plasmid elements (e.g. insertion of Ad sequences and/or a eukaryotic gene of interest linked to a ribosomal promoter), the plasmid sequences are referred to as the plasmid backbone.

The term "selectable marker" has used herein means an identifying factor, usually an antibiotic or chemical resistance gene, that is able to be selected for based upon the marker gene's effect, i.e., resistance to an antibiotic, resistance to a herbicide, colorimetric markers, enzymes, fluorescent markers, and the like, wherein the effect is used to track the inheritance of a nucleic acid of interest and/or to identify a cell or organism that has inherited the nucleic acid of interest. Examples of selectable marker genes known and used in the art include: genes providing resistance to ampicillin, streptomycin, gentamycin, kanamycin, hygromycin, bialaphos herbicide, sulfonamide, and the like; and genes that are used as phenotypic markers, i.e., anthocyanin regulatory genes, isopentanyl transferase gene, and the like.

Vectors may be introduced into the desired host cells by methods known in the art, e.g., transfection, electroporation, microinjection, transduction, cell fusion, DEAE dextran, calcium phosphate precipitation, lipofection (lysosome fusion), use of a gene gun, or a DNA vector transporter (see, e.g., Wu et al., , J. Biol. Chem. 1992. 267:963-967; Wu and Wu, J. Biol. Chem. 1988, 263:14621-14624; and Hartmut et al., Canadian Patent Application No. 2,012,311, filed March 15, 1990).

The term "transfection" means the uptake of exogenous or heterologous RNA or DNA by a host cell. A cell has been "transfected" by exogenous or heterologous RNA or DNA when such RNA or DNA has been introduced inside the host cell. A cell has been "transformed" by exogenous or heterologous RNA or DNA when the transfected RNA or DNA effects a phenotypic change. The transforming RNA or DNA can be integrated (covalently linked) into chromosomal DNA making up the genome of the cell.

"Transformation" refers to the transfer of a nucleic acid fragment into the genome of a host cell or organism, resulting in genetically stable inheritance. Host cells or organisms containing the transformed nucleic acid fragments are referred to as "transgenic" or "recombinant" or "transformed" organisms.

In addition, the vector of the invention comprising the cassette of the invention may include one or more origins for replication in the cellular hosts in which their amplification or their expression is sought, reporter genes or selectable markers.

In a particular embodiment, the vectors of the present invention may be episomal or integrating.

In a preferred embodiment, the vector of the invention comprises the genetic cassette which comprises the SEQ ID NO: 14. In another preferred embodiment, the vector of the invention comprises the genetic cassette which comprises the SEQ ID NO: 17. In a more preferred embodiment, the vector of the invention is selected from the SEQ ID NO: 18 or the SEQ ID NO: 19.

In addition to the aforementioned characteristics, the genetic cassette and the vectors of the present invention also allow for further studies, non-TRAP related, to be performed, since further embodiments of the present invention allow for the partial removal of the genetic cassette as well as targeted integration into the genome at the locus of the genetic cassette.

More particularly, the genetic cassette and the vectors of the present invention are useful:
(i) for generating host cells and/or non-human organisms,
(ii) for tissue-specific transcriptomics analysis,
(iii) to study gene function at various developmental stages,
(iv) as a model of animal and/or human diseases, or
(v) as a drug validation model in drug development.

A further aspect of the invention relates to a host cell, hereinafter host cell of the invention, comprising the genetic cassette of the invention and/or the vector of the invention.

The term "host cell" as used herein refers to a cell into which exogenous DNA has been introduced, either *in vitro, ex-vivo,* or *in vivo.* It should be understood that such terms are intended to refer not only to the particular subject cell, but, also to the progeny of such a cell. Because certain modifications may occur in succeeding generations due to either mutation or environmental influences, such progeny may not, in fact, be identical to the parent cell, but are still included within the scope of the term "host cell" as used herein. A host cell can be used, for example, for expression of a nucleic acid of interest, propagation of plasmid vectors and/or delivery of a sequence of interest to cells, wherein the nucleic acid of interest or plasmid vector are present episomally in these cells. The episomal vectors are not integrated into the host genome and are retained in self-replicating form in the host cell. The term episomally replicating is understood here as that the vector is not integrated to the genome of the host cell, but exists in parallel, is also replicated during the cell cycle and in the course of this the vector copies-depending on the number of the copies present before and after cell division-are distributed statistically in the resulting cells. Examples of such cells include, without limitation, bacterial cells like *Escherichia coli cells and Pichia pastoris cells, yeast cells like Saccharomyces cerevisiae and Schizosaccharomyces pombe, insect cells like Spodoptera frugiperda Sf21 and Sf19 cells, mammalian cells like human embryonal kidney cells, Chinese hamster ovary cells and Hella cells.* In addition, host cells can also be cells obtained by integrating the genetic cassette, or the vector of the invention, into their genome by known methods in the art. Preferably host cells include prokaryotic and eukaryotic cells selected from any of the kingdoms of life. Several cell types are known in the art and a skilful person in the subject matter will be able to identify an ideal cell type for use in the specific application. Preferred cells include, but are not limited to, mammalian cells, avian cells, amphibian cells, plant cells, fish cells, bacterial cells and insect cells. In a more preferred embodiment, the host cell is selected from a list comprising gram-negative bacteria cells, zebrafish cells, Drosophila cells, mouse cells and non-human cells.

A further aspect of the invention relates to a non-human organism, hereinafter the non-human organism of the invention, comprising the genetic cassette of the invention, the vector of the invention, or the host cell of the invention.

The term "organism" as used herein refers to a body carrying on processes of life, which has various properties, such as, representatively, cellular structure, proliferation (self-reproduction), growth, regulation, metabolism, repair ability, and the like. Typically, organisms possess basic attributes, such as heredity controlled by nucleic acids and proliferation in which metabolism controlled by proteins is involved. Organisms may include natural, wild-type, artificially manipulated, genetically modified, hybridized, or other variants or isolates. Organisms include viruses, prokaryotic organisms, eukaryotic organisms (e.g., unicellular organisms such as yeast, etc.) and multicellular organisms (e.g., plants, animals, etc.). It will be understood that, as the term is used herein, "organism" also refers to and encompasses cells as defined herein, and tissues derived from them, and that the methods of the present disclosure may be applied to any such cell or tissues.

In a specific embodiment, the non-human organism is a prokaryotic organism or a eukaryotic organism. In another specific embodiment, the non-human organism is an invertebrate organism or a vertebrate organism. Preferably, the non-human organism is selected from the group consisting of a bacterium, a fungus, a yeast, a nematode, an insect, a fish, a plant, a bird, an animal, and a mammal. More preferably, the non-human organism is a yeast, a nematode, an insect, a plant, a fish, an amphibian, a chicken, a hamster, a mouse, a rat, a rabbit, a cat, a dog, a bovine, a goat, a cow, a pig, a horse, a sheep, a simian, a monkey, or a chimpanzee. In a preferred embodiment, the animal is a rodent. In another preferred embodiment, the animal is a fish. In a more preferred embodiment, the animal is a zebrafish.

In another aspect, the invention comprises a kit and/or device comprising the genetic cassette, the vector, the host cell, the culture of host cell, the tissue, the non-human organism of the invention, or any combination thereof.

The kit of the invention comprises at least the genetic cassette of the invention or the vector of the invention having the genetic cassette, said genetic cassette having the necessary elements to allow its integration into the genome of a host cell. Said host cell may in addition also be contained in the kit. The kit further may contain a nucleic acid with transposase activity that allows the integration of the genetic cassette, or from the genetic cassette from the vector into the genome of the host cell. The kit may further include other components that find use in the subject invention, e.g., buffers, delivery vehicles, etc. The various components of the kit may be present in separate containers or certain compatible components may be combined into a single container, as desired. In addition to the above components, the subject kits will further include instructions for practicing the subject invention. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Yet another means would be a computer readable medium, e.g. a CD, an USB, etc., on which the information has been recorded. Yet another means that may be present is a website address which may be used via the internet to access the information at a removed site. Any convenient means may be present in the kits.

Another aspect of the present invention includes a method *in vitro* or *ex vivo* for producing a transgenic host cell and/or a transgenic non-human organism of the invention wherein the method comprises the insertion of the genetic cassette or the vector of the invention into a suitable cell and/or a suitable non-human organism.

Methods to produce a transgenic host cell or a transgenic non-human organism according to the present invention, preferably by using transposon-based nucleic acids such as vectors are well known in the art and several methodologies can be found in the literature (Miskey, C., et al. CMLS, Cell. Mol. Life Sci. (2005) 62: 629). The method of present invention may in addition contain other steps like culturing the non-human organism host cells into a fully developed non-human organism - the founder organism, screening for the reporter gene in the founder organism, outcrossing the founder organism with *wild type* non-human organism to generate F1 generation organism, screening the F1 generation for specific tissue labelling of the reporter gene, label of founder organism according to the specific reporter gene label detected in F1 generation, establish stable organism lines from founder organism.

A "transgenic non-human animal" refers to an animal, other than a human, that comprises a gene construct ("transgene") not found in a wild-type animal of the same species or breed. A "transgene" as referred to in this context has the normal meaning in the art of biotechnology and includes a genetic sequence which has been produced or altered by recombinant DNA or RNA technology and which has been introduced into an animal cell. The transgene may include genetic sequences derived from an animal cell, which may be of the same or different species or breed as the cell into which the transgene is introduced. Typically, the transgene has been introduced into the animal by human manipulation such as, for example, by transformation but any method can be used as one of skill in the art recognizes. In the present invention the transgene is the genetic cassette or the vector of the invention.

As it is mentioned previously, techniques for producing transgenic non-human animals are well known in the art. Transformation of a polynucleotide molecule into a cell can be accomplished by any method by which a polynucleotide molecule can be inserted into the cell. Transformation techniques include, but are not limited to, transfection, electroporation, microinjection, lipofection, adsorption, and cell fusion. A recombinant cell may remain unicellular or may grow into a tissue, organ or a multicellular organism. Transformed polynucleotide molecules can remain extrachromosomal or can integrate into one or more sites within a chromosome of the transformed (i.e., recombinant) cell in such a manner that their ability to be expressed is retained. In a preferred embodiment, the transformation technique is microinjection. In a most preferred method, however, the appropriate genetic cassette or vector of the invention are microinjected into the pronucleus or cytoplasm of embryos, preferably at the single cell stage, and the embryos allowed to develop into mature transgenic animals.

In another aspect, the present invention also relates to the use *in vitro* or *ex vivo* of the genetic cassette, or the vector of the invention for generating transgenic host cells and/or transgenic non-human organisms.

In another aspect, the present invention also relates to the use *in vitro* or *ex vivo* of the genetic cassette, the vector, the transgenic host cell, the transgenic non-human organism, or the kit according to the present invention,
(i) for tissue-specific transcriptomics analysis,
(ii) to study gene function at various developmental stages,
(iii) as an animal model of human disease, or
(iv) as a drug validation model in drug development.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### DESCRIPTION OF THE DRAWINGS

**Fig. 1****.** EGFP-rpl10a expression in the transgenic line TT15 at 35 hours post-fertilization (hpf) (lateral view) is limited to the lateral line system cells (Y = yolk; E =eye).
**Fig. 2** Schematic representation of the vectors used in the examples.
**Fig. 3****.** EGFP-rpl10a fussion gene expression profiles in different zebrafish transgenic lines: TT1 at 48hpf (A, A'), hindbrain (medulla oblongata and spinal cord, respectively); TT2 at 48 hpf (B), 72 hpf (B'), lenses; TT5 at 48hpf (C, C'), first branchial arch and jaw, respectively; TT6 at 48hpf (D), somites; TT7 at 48hpf (E), central nervous system; TT8 at 48hpf (F), 72hpf (F') olfactory bulb; TT9 at 48hpf (G) 72hpf (G') optic tectum; TT10 at 48hpf (H) neurons from dorsal root ganglion; TT11 at 48hpf (I), 72hpf (I') branchial arches, jaw and heart, respectively; TT13 at 48hpf (J) dorsal root ganglion; TT15 at 40hpf (K) lateral line system; TT16 at 48hpf (L), 72hpf (L'), mesencephalon; TT18 at 48 hpf (M) whole nervous system; TT21 at 48hpf (N), 72hpf (N') mesencephalon; TT25 at 48 hpf (O) 72 hpf (O') anterior nervous system; TT27 at 5dpf (P, P') telencephalon; TT28 at 48hpf (Q, Q') hindbrain (spinal cord, medulla oblongata, cerebellum) and fins, respectively; TT35 at 48hpf (R, R') telencephalon, eyes, respectively; TT37 at 48hpf (S, S') eyes; TT42 at 48 hpf (T, T') mesencephalon; TT46 at 48 hpf (U, U') telencephalon, mesencephalon, respectively; TT47 at 48hpf (V, V') endoderm, fins, archial branches, respectively; TT50 at 48hpf (W) spinal cord, somites; TT53 at 48hpf (X) 72 hpf (X') fore and midbrain, eyes, respectively; TT54 at 48hpf (Y, Y') fins, hindbrain, branchial arches, respectively; TT60 at 48 hpf (Z), notochord.

The following examples are offered by way of illustration and not by way of limitation. It is understood that the examples and embodiments described herein are for illustrative purposes only, and persons skilled in the art will recognize various reagents or parameters that can be altered without departing from the spirit of the invention or the scope of the appended claims.

### EXAMPLES

### Materials and Methods

### Genetic cassette and Vector construction

The vectors of the invention may be produced by standard methods of restriction enzyme cleavage, ligation and molecular cloning as known for the skilled person. Briefly, the vector of the invention named as Tol2_trap:TRAP (SEQ ID NO: 18) was created inserting the minimal promoter gata2p (SEQ ID NO: 13) to the commercial plasmid Tol2-zTRAP from Addgene (plasmid #42236). The gata2 promoter (SEQ ID NO: 13) was extracted from the ZED vector (Bessa, J. et al. Developmental dynamics. 2009, 238(9):2409-2417) using the restriction enzymes Sall and BamHI (1h at 37ºC). The commercial Tol2-zTRAP vector (Addgene) was also cut with these two restriction enzymes in the same conditions. Afterwards, both elements were ligated using the T4 DNA ligase enzyme, incubating overnight at 16ºC. The next day, *Escherichia coli* competent cells (DH5α) were transformed with the ligation and plated and cultured in LB agar with ampicillin. To select the correctly ligated plasmids, bacterial cultures were prepared in liquid LB from single colonies picked from the plate. The plasmids were isolated from each culture and analysed with a restriction assay using the Nhel enzyme. The correct plasmid was purified with a plasmid miniprep kit (GenElute™, Sigma-Aldrich) and its concentration measured by spectrophotometry (Nanodrop One, Thermo Fisher). Further components could be added to the vector/plasmid using similar methods.

### EGFP Zebrafish Transgenic lines generation

The zebrafish transgenic lines are generated through the insertion of the vector of the invention in the host cells by microinjection of one-cell stage embryos. Briefly, the vector of the invention Tol2_trap:TRAP (SEQ ID NO: 18) carrying the genetic cassette of the invention (SEQ ID NO: 14) can be used. In order to obtain one-cell stage embryos, male and female adult zebrafishes are separated the day before. At the moment of the microinjection they are put together in a breeding tank. After the embryos have been laid, the zebrafishes are returned to their original tank and the embryos are collected using a strainer. The embryos are placed in a petri dish containing saline solution, ready to be microinjected. For this, a needle glass attached to a micromanipulator is used. The micromanipulator is necessary to control the needle and change its position in an easy way. The needle is filled with a solution containing 25ng/µL of the purified vector of the invention (SEQ ID NO: 18) and 25ng/µL of the Tol2 transposase mRNA (SEQ ID NO: 20). Each embryo in one-cell stage is microinjected with 5nL of the solution. Afterwards the embryos are kept at 28ºC and 24 hours post-fertilization (hpf) they are examined using a fluorescence micro stereoscope in search of EGFP-rpl10a expression (seen as green fluorescence). All the positive embryos were selected and raised. For this, they were placed in a petri dish at a maximum number of 100 embryos per plate. 5 days after fertilization the larvae were transferred to regular tanks and fed. During the first few days they were kept in around 3 cm of water, increasing its volume gradually each 2 or 3 days until the larvae are 3 weeks old and they can be in the water system flow. When fishes are adult and sexually mature (around 3 months old) they can breed.

From this moment, the grown microinjected fishes are crossed one by one with wild-type ones (outcross). The embryos of each cross are collected and screened for GFP expression at 8, 24, 48 and 72 hpf using a fluorescence micro stereoscope. If less than 150 embryos were obtained from an outcross, an additional one was set up in order to get enough embryos for the screening. The fishes that transmitted GFP expression to their progeny were considered as positives (founders) and labelled with a number that designated the line of which the fish was founder. The embryos that showed tissue-specific GFP expression were raised to establish and maintain each transgenic line.

### Imaging

To characterize the GFP expression pattern of each transgenic line, pictures of the embryos were taken at different developmental stages. For this, each founder fish was crossed with a wild-type one and the collected embryos were kept at 28ºC. At the moment of taking the photographs, the embryos were dechorionated with forceps if needed and mounted in 2% methylcellulose. The photographs were taken at 75% epiboly (8hpf), 24 hpf, 48 hpf and 72 hpf, using a camera Nikon and an Olympus fluorescence micro stereoscope.

### Example 1 - Transformation efficiency and germline transmission.

One-cell stage zebrafish embryos were injected with the composition comprising the vector of the invention comprising the genetic cassette of the invention shown in Fig. 1A and the Tol2 transposase expressing mRNA, as explained previously in material and methods. The embryos that presented some type of fluorescence were selected to be raised to adulthood using the established and standard husbandry practices commonly known in the state of the art (Lawrence C. Aquaculture. 2007. Vol.269 (1-4), Pag 1-20).

A total of 300 embryos reached adulthood. These embryos were then screened as described above for the marker gene, EGFP fluorescence. From the adult zebrafishes screened, 53 zebrafishes (approx. 17.5%) were positive for the marker gene, showing fluorescence signal of some kind. From these, 33 (approx. 11%) showed tissue-specific EGFP expression, i.e., expression of the marker gene constrained to a specific and delimited region in the zebrafish.

The results indicated that the efficiency of transformation was of 17.5% Furthermore, the results also indicate that the integration of the genetic cassette was unbiased and occurred randomly as demonstrated by the diversity of tissues presenting detectable EGFP expression, regardless of the germ layer from which the tissues derive or the tissue position along the embryo axes (see Example 2).

### Example 2 - Transgenic lines' characterization.

Embryos from each tissue-specific transgenic line generated were examined with a fluorescent micro stereoscope. Moreover, in order to keep record of the peculiarities of each line, photographs were taken at different stages of development.

The Fig. 3 shows images of the obtained lines having different expression patterns. The obtained transgenic lines has been identified with the letters "TT" followed by a number: TT1 at 48hpf (A, A'), hindbrain (medulla oblongata and spinal cord, respectively); TT2 at 48 hpf (B), 72 hpf (B'), lenses; TT5 at 48hpf (C, C'), first branchial arch and jaw, respectively; TT6 at 48hpf (D), somites; TT7 at 48hpf (E), central nervous system; TT8 at 48hpf (F), 72hpf (F') olfactory bulb; TT9 at 48hpf (G) 72hpf (G') optic tectum; TT10 at 48hpf (H) neurons from dorsal root ganglion; TT11 at 48hpf (I), 72hpf (I') branchial arches, jaw and heart, respectively; TT13 at 48hpf (J) dorsal root ganglion; TT15 at 40hpf (K) lateral line system; TT16 at 48hpf (L), 72hpf (L'), mesencephalon; TT18 at 48 hpf (M) whole nervous system; TT21 at 48hpf (N), 72hpf (N') mesencephalon; TT25 at 48 hpf (O) 72 hpf (O') anterior nervous system; TT27 at 5dpf (P, P') telencephalon; TT28 at 48hpf (Q, Q') hindbrain (spinal cord, medulla oblongata, cerebellum) and fins, respectively; TT35 at 48hpf (R, R') telencephalon, eyes, respectively; TT37 at 48hpf (S, S') eyes; TT42 at 48 hpf (T, T') mesencephalon; TT46 at 48 hpf (U, U') telencephalon, mesencephalon, respectively; TT47 at 48hpf (V, V') endoderm, fins, archial branches, respectively; TT50 at 48hpf (W) spinal cord, somites; TT53 at 48hpf (X) 72 hpf (X') fore and midbrain, eyes, respectively; TT54 at 48hpf (Y, Y') fins, hindbrain, branchial arches, respectively; TT60 at 48 hpf (Z), notochord.

### Conclusions

To date, we have identified 53 founders (zebrafish transgenic lines) that showed EGFP-rpl10a expression. Out of them, 33 showed specific expression patterns (24 lines showed in Fig. 3). These transgenic lines show very specific and unique expression patterns, some of them not previously described (TT5, TT15 or TT21). Therefore, the results show in the present invention indicate that the cassettes and the vectors of the invention in combination with the associated technology will constitute an improved tool for the dynamic analysis of tissue-specific transcriptomes. These unique transgenic lines will constitute powerful experimental tools for groups interested in the development and the physiology of the concerned tissues. Given the very general applicability of RNA-seq studies in biomedicine, and the sustained growth of model systems for the study of animal and/or human diseases, it is believed that the present invention has an important translational applicability. In the specific case of zebrafish, the systematic generation of transgenic lines should be relevant not only in developmental genetics, but also in physiology, toxicology and biomedical studies. Thus, the long-term economic and medical impact of developing these methods should not be underestimated.

## Claims

1. A genetic cassette comprising
a. a pair of transposase binding sites delimiting said genetic cassette, and
b. a fusion gene comprising
i. a reporter gene, and
ii. a ribosomal gene
wherein the fusion gene is in operational combination with a minimal promoter.

2. The genetic cassette according to claim 1 wherein the pair of transposase binding sites are recognized by a transposase selected from Sleeping Beauty or Tol2, wherein the Sleeping Beauty transposase comprises the SEQ ID NO: 2 and the Tol2 transposase comprises the SEQ ID NO: 3.

3. The genetic cassette according to claim 2 wherein the pair of transposase binding sites is recognized by Tol2 transposase and comprising the SEQ ID NO: 4 and the SEQ ID NO: 5.

4. The genetic cassette according to any of claims 1 to 3 wherein the reporter gene is selected from the list consisting of: luciferase (Luc), green fluorescent protein (GFP), enhanced green fluorescent protein (EGFP) red fluorescent protein (RFP), Cyan fluorescent protein (CFP), Yellow fluorescent protein (YFP), chloramphenicol acetyltransferase (CAT), β-galactosidase (LacZ) and β-glucuronidase (Gus), preferably the reporter gene is EGFP wherein the EGFP comprises the SEQ ID NO: 6.

5. The genetic cassette according to any of claims 1 to 4 wherein the ribosomal gene is a large ribosomal subunit protein gene or a small ribosomal subunit protein gene, preferably, the ribosomal gene is the ribosomal protein L10a gene comprising the SEQ ID NO: 8 or SEQ ID NO: 10.

6. The genetic cassette according to any of claims 1 to 5 wherein the minimal promoter is selected from the list consisting of: ef1a, gata1p, gata2p, keratin8, keratin4, hsp70, c-fos, E1b, thymidine kinase, carp β-actin, Hspa1a, preferably, the minimal promoter is the gata2p comprising the SEQ ID NO: 13.

7. The genetic cassette according to any of claims 1 to 6 further comprising a pair of Cre recombinase recognition sites, wherein the first Cre recombinase recognition site is located upstream of the minimal promoter and the second Cre recombinase recognition site is located downstream of the fusion gene, preferably is a LoxP site comprising the SEQ ID NO: 15.

8. The genetic cassette according to any of claims 1 to 7 further comprising an integrase specific site, preferably an attP site comprises the SEQ ID NO: 16.

9. A genetic cassette according to any of claims 1 to 8 **characterized in that** comprises the SEQ ID NO: 14 or SEQ ID NO: 17.

10. A vector comprising the genetic cassette according to any of claims 1 to 9.

11. A host cell, a culture of host cell, a tissue or a non-human organism comprising the genetic cassette according to any of claims 1 to 9 or the vector according to claim 10.

12. A kit comprising the genetic cassette according to any of claims 1 to 9, the vector according to claim 10, the host cell, the culture of host cell, the tissue or the non-human organism according to claim 11, or any combination thereof.

13. An *in vitro* or *ex vivo* method for producing the host cell, the culture of host cell, the tissue or the non-human organism of claim 11, which comprises the insertion of the genetic cassette according to any of claims 1 to 9, or the vector according to claim 10, into a suitable cell.

14. Use *in vitro* or *ex vivo* of the genetic cassette according to any of claims 1 to 9 or the vector according to claim 10, for generating host cells, culture of host cell, tissue and/or non-human organisms.

15. Use *in vitro* or *ex vivo* of the genetic cassette according to any of claims 1 to 9, the vector according to claim 10, the host cell, the culture of host cell, the tissue and/or the non-human organism according to claim 11, or the kit according to claim 12
(i) for tissue-specific transcriptomics analysis,
(ii) to study gene function at various developmental stages,
(iii) as an animal model of animal and/or human disease, or
(iv) as a drug validation model in drug development.


SEQUENCE LISTING

<110>  CSIC - Consejo Superior de Investigaciones Científicas

<120>  Vectors for tissue specific transcriptomics

<130>  EP1641.1470

<160>  20

<170>  PatentIn version 3.5

<210>  1
<211>  192
<212>  DNA
<213>  Artificial Sequence

<220>
<223>  PolyA sequence

<400>  1
cagacatgat aagatacatt gatgagtttg gacaaaccac aactagaatg cagtgaaaaa      60

aatgctttat ttgtgaaatt tgtgatgcta ttgctttatt tgtaaccatt ataagctgca     120

ataaacaagt taacaacaac aattgcattc attttatgtt tcaggttcag ggggaggtgt     180

gggaggtttt tt                                                         192


<210>  2
<211>  140
<212>  PRT
<213>  Oncorhynchus mykiss

<400>  2

Met Gly Lys Ser Lys Glu Lys Ser Gly Ser Ser Leu Gly Ala Ile Ser
1               5                   10                  15


Lys His Leu Lys Val Pro Cys Ser Ser Val Gln Ile Ile Val Arg Lys
            20                  25                  30


Tyr Lys His His Gly Thr Thr Gln Pro Ser Tyr Arg Ser Gly Arg Arg
        35                  40                  45


Arg Val Leu Ser Pro Arg Asp Glu Arg Thr Leu Val Arg Lys Val Gln
    50                  55                  60


Ile Asn Pro Arg Thr Thr Ala Lys Asp Leu Val Lys Met Leu Glu Glu
65                  70                  75                  80


Thr Gly Thr Lys Val Ser Ile Ser Thr Val Lys Arg Val Leu Tyr Arg
                85                  90                  95


His Asn Leu Lys Gly Arg Ser Ala Arg Lys Lys Pro Leu Leu Gln Asn
            100                 105                 110

Arg His Lys Lys Ala Arg Leu Gln Phe Ala Thr Ala His Gly Asp Lys
        115                 120                 125

Asp Arg Thr Phe Leu Glu Lys Cys Phe Leu Val Leu
    130                 135                 140

<210>  3
<211>  649
<212>  PRT
<213>  Oryzias latipes

<400>  3

Met Glu Glu Val Cys Asp Ser Ser Ala Ala Ala Ser Ser Thr Val Gln
1               5                   10                  15

Asn Gln Pro Gln Asp Gln Glu His Pro Trp Pro Tyr Leu Arg Glu Phe
            20                  25                  30

Phe Ser Leu Ser Gly Val Asn Lys Asp Ser Phe Lys Met Lys Cys Val
        35                  40                  45

Leu Cys Leu Pro Leu Asn Lys Glu Ile Ser Ala Phe Lys Ser Ser Pro
    50                  55                  60

Ser Asn Leu Arg Lys His Ile Glu Arg Met His Pro Asn Tyr Leu Lys
65                  70                  75                  80

Asn Tyr Ser Lys Leu Thr Ala Gln Lys Arg Lys Ile Gly Thr Ser Thr
                85                  90                  95

His Ala Ser Ser Ser Lys Gln Leu Lys Val Asp Ser Val Phe Pro Val
            100                 105                 110

Lys His Val Ser Pro Val Thr Val Asn Lys Ala Ile Leu Arg Tyr Ile
        115                 120                 125

Ile Gln Gly Leu His Pro Phe Ser Thr Val Asp Leu Pro Ser Phe Lys
    130                 135                 140

Glu Leu Ile Ser Thr Leu Gln Pro Gly Ile Ser Val Ile Thr Arg Pro
145                 150                 155                 160

Thr Leu Arg Ser Lys Ile Ala Glu Ala Ala Leu Ile Met Lys Gln Lys
                165                 170                 175

Val Thr Ala Ala Met Ser Glu Val Glu Trp Ile Ala Thr Thr Thr Asp
            180                 185                 190

Cys Trp Thr Ala Arg Arg Lys Ser Phe Ile Gly Val Thr Ala His Trp
        195                 200                 205

Ile Asn Pro Gly Ser Leu Glu Arg His Ser Ala Ala Leu Ala Cys Lys
    210                 215                 220

Arg Leu Met Gly Ser His Thr Phe Glu Val Leu Ala Ser Ala Met Asn
225                 230                 235                 240

Asp Ile His Ser Glu Tyr Glu Ile Arg Asp Lys Val Val Cys Thr Thr
                245                 250                 255

Thr Asp Ser Gly Ser Asn Phe Met Lys Ala Phe Arg Val Phe Gly Val
            260                 265                 270

Glu Asn Asn Asp Ile Glu Thr Glu Ala Arg Arg Cys Glu Ser Asp Asp
        275                 280                 285

Thr Asp Ser Glu Gly Cys Gly Glu Gly Ser Asp Gly Val Glu Phe Gln
    290                 295                 300

Asp Ala Ser Arg Val Leu Asp Gln Asp Asp Gly Phe Glu Phe Gln Leu
305                 310                 315                 320

Pro Lys His Gln Lys Cys Ala Cys His Leu Leu Asn Leu Val Ser Ser
                325                 330                 335

Val Asp Ala Gln Lys Ala Leu Ser Asn Glu His Tyr Lys Lys Leu Tyr
            340                 345                 350

Arg Ser Val Phe Gly Lys Cys Gln Ala Leu Trp Asn Lys Ser Ser Arg
        355                 360                 365

Ser Ala Leu Ala Ala Glu Ala Val Glu Ser Glu Ser Arg Leu Gln Leu
    370                 375                 380

Leu Arg Pro Asn Gln Thr Arg Trp Asn Ser Thr Phe Met Ala Val Asp
385                 390                 395                 400

Arg Ile Leu Gln Ile Cys Lys Glu Ala Gly Glu Gly Ala Leu Arg Asn
                405                 410                 415

Ile Cys Thr Ser Leu Glu Val Pro Met Phe Asn Pro Ala Glu Met Leu
            420                 425                 430

Phe Leu Thr Glu Trp Ala Asn Thr Met Arg Pro Val Ala Lys Val Leu

435                 440                 445

Asp Ile Leu Gln Ala Glu Thr Asn Thr Gln Leu Gly Trp Leu Leu Pro
    450                 455                 460

Ser Val His Gln Leu Ser Leu Lys Leu Gln Arg Leu His His Ser Leu
465                 470                 475                 480

Arg Tyr Cys Asp Pro Leu Val Asp Ala Leu Gln Gln Gly Ile Gln Thr
                485                 490                 495

Arg Phe Lys His Met Phe Glu Asp Pro Glu Ile Ile Ala Ala Ala Ile
            500                 505                 510

Leu Leu Pro Lys Phe Arg Thr Ser Trp Thr Asn Asp Glu Thr Ile Ile
        515                 520                 525

Lys Arg Gly Met Asp Tyr Ile Arg Val His Leu Glu Pro Leu Asp His
    530                 535                 540

Lys Lys Glu Leu Ala Asn Ser Ser Ser Asp Asp Glu Asp Phe Phe Ala
545                 550                 555                 560

Ser Leu Lys Pro Thr Thr His Glu Ala Ser Lys Glu Leu Asp Gly Tyr
                565                 570                 575

Leu Ala Cys Val Ser Asp Thr Arg Glu Ser Leu Leu Thr Phe Pro Ala
            580                 585                 590

Ile Cys Ser Leu Ser Ile Lys Thr Asn Thr Pro Leu Pro Ala Ser Ala
        595                 600                 605

Ala Cys Glu Arg Leu Phe Ser Thr Ala Gly Leu Leu Phe Ser Pro Lys
    610                 615                 620

Arg Ala Arg Leu Asp Thr Asn Asn Phe Glu Asn Gln Leu Leu Leu Lys
625                 630                 635                 640

Leu Asn Leu Arg Phe Tyr Asn Phe Glu
                645

<210>  4
<211>  148
<212>  DNA
<213>  Artificial Sequence

<220>
<223>  First Tol2 transposase recognition sequence

<400>  4
cagaggtgta aaaagtactc aaaaatttta ctcaagtgaa agtacaagta cttagggaaa      60

attttactca attaaaagta aaagtatctg gctagaatct tacttgagta aaagtaaaaa     120

agtactccat taaaattgta cttgagta                                        148


<210>  5
<211>  199
<212>  DNA
<213>  Artificial Sequence

<220>
<223>  Second Tol2 transposase recognition sequence

<400>  5
aagtgatctc caaaaaataa gtactttttg actgtaaata aaattgtaag gagtaaaaag      60

tacttttttt tctaaaaaaa tgtaattaag taaaagtaaa agtattgatt tttaattgta     120

ctcaagtaaa gtaaaaatcc ccaaaaataa tacttaagta cagtaatcaa gtaaaattac     180

tcaagtactt tacacctct                                                  199


<210>  6
<211>  717
<212>  DNA
<213>  Artificial Sequence

<220>
<223>  EGFP


<220>
<221>  CDS
<222>  (1)..(717)

<400>  6
atg gtg agc aag ggc gag gag ctg ttc acc ggg gtg gtg ccc atc ctg       48
Met Val Ser Lys Gly Glu Glu Leu Phe Thr Gly Val Val Pro Ile Leu
1               5                   10                  15

gtc gag ctg gac ggc gac gta aac ggc cac aag ttc agc gtg tcc ggc       96
Val Glu Leu Asp Gly Asp Val Asn Gly His Lys Phe Ser Val Ser Gly
            20                  25                  30

gag ggc gag ggc gat gcc acc tac ggc aag ctg acc ctg aag ttc atc      144
Glu Gly Glu Gly Asp Ala Thr Tyr Gly Lys Leu Thr Leu Lys Phe Ile
        35                  40                  45

tgc acc acc ggc aag ctg ccc gtg ccc tgg ccc acc ctc gtg acc acc      192
Cys Thr Thr Gly Lys Leu Pro Val Pro Trp Pro Thr Leu Val Thr Thr
    50                  55                  60

ctg acc tac ggc gtg cag tgc ttc agc cgc tac ccc gac cac atg aag      240
Leu Thr Tyr Gly Val Gln Cys Phe Ser Arg Tyr Pro Asp His Met Lys
65                  70                  75                  80

cag cac gac ttc ttc aag tcc gcc atg ccc gaa ggc tac gtc cag gag      288
Gln His Asp Phe Phe Lys Ser Ala Met Pro Glu Gly Tyr Val Gln Glu
                85                  90                  95

cgc acc atc ttc ttc aag gac gac ggc aac tac aag acc cgc gcc gag 336
Arg Thr Ile Phe Phe Lys Asp Asp Gly Asn Tyr Lys Thr Arg Ala Glu
            100                 105                 110

gtg aag ttc gag ggc gac acc ctg gtg aac cgc atc gag ctg aag ggc 384
Val Lys Phe Glu Gly Asp Thr Leu Val Asn Arg Ile Glu Leu Lys Gly
        115                 120                 125

atc gac ttc aag gag gac ggc aac atc ctg ggg cac aag ctg gag tac 432
Ile Asp Phe Lys Glu Asp Gly Asn Ile Leu Gly His Lys Leu Glu Tyr
    130                 135                 140

aac tac aac agc cac aac gtc tat atc atg gcc gac aag cag aag aac 480
Asn Tyr Asn Ser His Asn Val Tyr Ile Met Ala Asp Lys Gln Lys Asn
145                 150                 155                 160

ggc atc aag gtg aac ttc aag atc cgc cac aac atc gag gac ggc agc 528
Gly Ile Lys Val Asn Phe Lys Ile Arg His Asn Ile Glu Asp Gly Ser
                165                 170                 175

gtg cag ctc gcc gac cac tac cag cag aac acc ccc atc ggc gac ggc 576
Val Gln Leu Ala Asp His Tyr Gln Gln Asn Thr Pro Ile Gly Asp Gly
            180                 185                 190

ccc gtg ctg ctg ccc gac aac cac tac ctg agc acc cag tcc gcc ctg 624
Pro Val Leu Leu Pro Asp Asn His Tyr Leu Ser Thr Gln Ser Ala Leu
        195                 200                 205

agc aaa gac ccc aac gag aag cgc gat cac atg gtc ctg ctg gag ttc 672
Ser Lys Asp Pro Asn Glu Lys Arg Asp His Met Val Leu Leu Glu Phe
    210                 215                 220

gtg acc gcc gcc ggg atc act ctc ggc atg gac gag ctg tac aag 717
Val Thr Ala Ala Gly Ile Thr Leu Gly Met Asp Glu Leu Tyr Lys
225                 230                 235


<210> 7
<211> 239
<212> PRT
<213> Artificial Sequence

<220>
<223> Synthetic Construct

<400> 7

Met Val Ser Lys Gly Glu Glu Leu Phe Thr Gly Val Val Pro Ile Leu
1               5                   10                  15


Val Glu Leu Asp Gly Asp Val Asn Gly His Lys Phe Ser Val Ser Gly
            20                  25                  30


Glu Gly Glu Gly Asp Ala Thr Tyr Gly Lys Leu Thr Leu Lys Phe Ile
        35                  40                  45


Cys Thr Thr Gly Lys Leu Pro Val Pro Trp Pro Thr Leu Val Thr Thr
    50                  55                  60

Leu Thr Tyr Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys
225                 230                 235

<210>  8
<211>  654
<212>  DNA
<213>  Mus musculus

<220>
<221>  CDS
<222>  (1)..(654)

<400>  8
atg agc agc aaa gtc tca cgc gac acc ctg tac gag gcg gtg cgg gaa      48
Met Ser Ser Lys Val Ser Arg Asp Thr Leu Tyr Glu Ala Val Arg Glu
1               5                   10                  15

gtc ctg cac ggg aac cag cgc aag cgc cgc aag ttt ctg gag acg gtg      96
Val Leu His Gly Asn Gln Arg Lys Arg Arg Lys Phe Leu Glu Thr Val
            20                  25                  30

gag ctg cag atc agc ctg aag aac tac gac cct cag aag gac aaa cgt     144
Glu Leu Gln Ile Ser Leu Lys Asn Tyr Asp Pro Gln Lys Asp Lys Arg
        35                  40                  45

ttc tcg ggc acc gtc agg ctc aag tcc acc cca cgc ccc aag ttc tcg     192
Phe Ser Gly Thr Val Arg Leu Lys Ser Thr Pro Arg Pro Lys Phe Ser
    50                  55                  60

gtg tgc gtt ctg ggg gac cag cag cac tgt gat gaa gcc aag gcc gtg     240
Val Cys Val Leu Gly Asp Gln Gln His Cys Asp Glu Ala Lys Ala Val
65                  70                  75                  80

gat atc ccc cac atg gac atc gag gcg ctc aag aag ctt aac aaa aac     288
Asp Ile Pro His Met Asp Ile Glu Ala Leu Lys Lys Leu Asn Lys Asn
                85                  90                  95

aag aag ttg gtc aag aag ctg gct aag aag tac gat gcc ttt ttg gcc     336
Lys Lys Leu Val Lys Lys Leu Ala Lys Lys Tyr Asp Ala Phe Leu Ala
            100                 105                 110

tct gag tct ctg att aag cag atc cca cgt atc ctg ggc cca ggc cta     384
Ser Glu Ser Leu Ile Lys Gln Ile Pro Arg Ile Leu Gly Pro Gly Leu
        115                 120                 125

aac aag gct ggc aag ttc ccc tcc ctg ctg aca cac aat gaa aac atg     432
Asn Lys Ala Gly Lys Phe Pro Ser Leu Leu Thr His Asn Glu Asn Met
    130                 135                 140

gtg gcc aaa gtg gat gag gtg aaa tcg aca atc aag ttc cag atg aag     480
Val Ala Lys Val Asp Glu Val Lys Ser Thr Ile Lys Phe Gln Met Lys
145                 150                 155                 160

aag gtg ctg tgt ttg gcc gtc gct gtt ggc cac gtg aag atg acc gat     528
Lys Val Leu Cys Leu Ala Val Ala Val Gly His Val Lys Met Thr Asp
                165                 170                 175

gat gag cta gtc tac aac att cat ctg gct gtc aat ttc ttg gtg tcc     576
Asp Glu Leu Val Tyr Asn Ile His Leu Ala Val Asn Phe Leu Val Ser
            180                 185                 190

ttg ctt aag aaa aac tgg caa aac gtg cgg gct ctg tac atc aag agc     624
Leu Leu Lys Lys Asn Trp Gln Asn Val Arg Ala Leu Tyr Ile Lys Ser
        195                 200                 205

acc atg ggc aag ccc cag cgt ctg tat tag                             654
Thr Met Gly Lys Pro Gln Arg Leu Tyr
    210                 215

<210>  9
<211>  217
<212>  PRT
<213>  Mus musculus

<400>  9

Met Ser Ser Lys Val Ser Arg Asp Thr Leu Tyr Glu Ala Val Arg Glu
1               5                   10                  15

Val Leu His Gly Asn Gln Arg Lys Arg Arg Lys Phe Leu Glu Thr Val
            20                  25                  30

Glu Leu Gln Ile Ser Leu Lys Asn Tyr Asp Pro Gln Lys Asp Lys Arg
        35                  40                  45

Phe Ser Gly Thr Val Arg Leu Lys Ser Thr Pro Arg Pro Lys Phe Ser
    50                  55                  60

Val Cys Val Leu Gly Asp Gln Gln His Cys Asp Glu Ala Lys Ala Val
65                  70                  75                  80

Asp Ile Pro His Met Asp Ile Glu Ala Leu Lys Lys Leu Asn Lys Asn
                85                  90                  95

Lys Lys Leu Val Lys Lys Leu Ala Lys Lys Tyr Asp Ala Phe Leu Ala
            100                 105                 110

Ser Glu Ser Leu Ile Lys Gln Ile Pro Arg Ile Leu Gly Pro Gly Leu
        115                 120                 125

Asn Lys Ala Gly Lys Phe Pro Ser Leu Leu Thr His Asn Glu Asn Met
    130                 135                 140

Val Ala Lys Val Asp Glu Val Lys Ser Thr Ile Lys Phe Gln Met Lys
145                 150                 155                 160

Lys Val Leu Cys Leu Ala Val Ala Val Gly His Val Lys Met Thr Asp
                165                 170                 175

Asp Glu Leu Val Tyr Asn Ile His Leu Ala Val Asn Phe Leu Val Ser
            180                 185                 190

Leu Leu Lys Lys Asn Trp Gln Asn Val Arg Ala Leu Tyr Ile Lys Ser
        195                 200                 205

Thr Met Gly Lys Pro Gln Arg Leu Tyr
    210                 215

<210>  10
<211>  651
<212>  DNA
<213>  Danio rerio

<220>
<221>  CDS
<222>  (1)..(651)

<400>  10
atg agc aag gtc tcg aga gac acg ttg tac gag gcc gtt aag gag gtc        48
Met Ser Lys Val Ser Arg Asp Thr Leu Tyr Glu Ala Val Lys Glu Val

1                   5                   10                  15

cag gcc ggt tcc cgt cgc aag aag aga aag ttt ctt gaa acc gtg gaa          96
Gln Ala Gly Ser Arg Arg Lys Lys Arg Lys Phe Leu Glu Thr Val Glu
            20                  25                  30

ctc cag atc agc ttg aag aac tac gac cct cag aag gac aag cgt ttc          144
Leu Gln Ile Ser Leu Lys Asn Tyr Asp Pro Gln Lys Asp Lys Arg Phe
        35                  40                  45

tca ggc act gtc agg ctg aag acc acc ccg cgg ccc aag ttc tct gtc          192
Ser Gly Thr Val Arg Leu Lys Thr Thr Pro Arg Pro Lys Phe Ser Val
    50                  55                  60

tgc gtt ctt gga gac cag cag cat tgt gat gaa gca aag gcc gct gaa          240
Cys Val Leu Gly Asp Gln Gln His Cys Asp Glu Ala Lys Ala Ala Glu
65                  70                  75                  80

ctg cca cac atg gac att gag gct ctt aag aag ctc aat aag aac aag          288
Leu Pro His Met Asp Ile Glu Ala Leu Lys Lys Leu Asn Lys Asn Lys
                85                  90                  95

aag ctg gtc aag aag ctg gca aag aag tac gat gct ttc ctg gcc tct          336
Lys Leu Val Lys Lys Leu Ala Lys Lys Tyr Asp Ala Phe Leu Ala Ser
            100                 105                 110

gag tct ctg atc aag cag att cct cgt atc ctg ggc cct ggc ctc aac          384
Glu Ser Leu Ile Lys Gln Ile Pro Arg Ile Leu Gly Pro Gly Leu Asn
        115                 120                 125

aag gca gga aag ttt ccc tct ctg ctc acc cat aat gag aac ctg ggc          432
Lys Ala Gly Lys Phe Pro Ser Leu Leu Thr His Asn Glu Asn Leu Gly
    130                 135                 140

acc aag gtg gat gag gtg aaa tcc acc atc aaa ttc cag atg aag aag          480
Thr Lys Val Asp Glu Val Lys Ser Thr Ile Lys Phe Gln Met Lys Lys
145                 150                 155                 160

gtt ctg tgt ctg gct gtg gcc gtg ggt cat gtg aag atg agt gag gaa          528
Val Leu Cys Leu Ala Val Ala Val Gly His Val Lys Met Ser Glu Glu
                165                 170                 175

gag ctg gtc tac aac atc cac ctg gct gtc aac ttc ctg gtg tct ctg          576
Glu Leu Val Tyr Asn Ile His Leu Ala Val Asn Phe Leu Val Ser Leu
            180                 185                 190

ctg aag aag aac tgg caa aac gtc aga gct ctt tac atc aag agc acc          624
Leu Lys Lys Asn Trp Gln Asn Val Arg Ala Leu Tyr Ile Lys Ser Thr
        195                 200                 205

atg gga aaa cca cag cgc ctc tac tag                                      651
Met Gly Lys Pro Gln Arg Leu Tyr
    210                 215

<210>  11
<211>  216
<212>  PRT
<213>  Danio rerio

<400>  11

Met Ser Lys Val Ser Arg Asp Thr Leu Tyr Glu Ala Val Lys Glu Val

1               5                   10                  15

Gln Ala Gly Ser Arg Arg Lys Lys Arg Lys Phe Leu Glu Thr Val Glu
            20                  25                  30

Leu Gln Ile Ser Leu Lys Asn Tyr Asp Pro Gln Lys Asp Lys Arg Phe
        35                  40                  45

Ser Gly Thr Val Arg Leu Lys Thr Thr Pro Arg Pro Lys Phe Ser Val
    50                  55                  60

Cys Val Leu Gly Asp Gln Gln His Cys Asp Glu Ala Lys Ala Ala Glu
65                  70                  75                  80

Leu Pro His Met Asp Ile Glu Ala Leu Lys Lys Leu Asn Lys Asn Lys
                85                  90                  95

Lys Leu Val Lys Lys Leu Ala Lys Lys Tyr Asp Ala Phe Leu Ala Ser
            100                 105                 110

Glu Ser Leu Ile Lys Gln Ile Pro Arg Ile Leu Gly Pro Gly Leu Asn
        115                 120                 125

Lys Ala Gly Lys Phe Pro Ser Leu Leu Thr His Asn Glu Asn Leu Gly
    130                 135                 140

Thr Lys Val Asp Glu Val Lys Ser Thr Ile Lys Phe Gln Met Lys Lys
145                 150                 155                 160

Val Leu Cys Leu Ala Val Ala Val Gly His Val Lys Met Ser Glu Glu
                165                 170                 175

Glu Leu Val Tyr Asn Ile His Leu Ala Val Asn Phe Leu Val Ser Leu
            180                 185                 190

Leu Lys Lys Asn Trp Gln Asn Val Arg Ala Leu Tyr Ile Lys Ser Thr
        195                 200                 205

Met Gly Lys Pro Gln Arg Leu Tyr
    210                 215

<210>  12
<211>  1404
<212>  DNA
<213>  Artificial Sequence

<220>
<223>  Fusion gene of eGFP with the ribosomal protein L10 of Danio rerio

<400> 12
atggtgagca agggcgagga gctgttcacc ggggtggtgc ccatcctggt cgagctggac 60

ggcgacgtaa acggccacaa gttcagcgtg tccggcgagg gcgagggcga tgccacctac 120

ggcaagctga ccctgaagtt catctgcacc accggcaagc tgcccgtgcc ctggcccacc 180

ctcgtgacca ccctgaccta cggcgtgcag tgcttcagcc gctaccccga ccacatgaag 240

cagcacgact tcttcaagtc cgccatgccc gaaggctacg tccaggagcg caccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaactacaa cagccacaac gtctatatca tggccgacaa gcagaagaac 480

ggcatcaagg tgaacttcaa gatccgccac aacatcgagg acggcagcgt gcagctcgcc 540

gaccactacc agcagaacac ccccatcggc gacggccccg tgctgctgcc cgacaaccac 600

tacctgagca cccagtccgc cctgagcaaa gaccccaacg agaagcgcga tcacatggtc 660

ctgctggagt tcgtgaccgc cgccgggatc actctcggca tggacgagct gtacaagtcc 720

ggccggactc agatctcgag ctcaagcttc gaattcagca aggtctcgag agacacgttg 780

tacgaggccg ttaaggaggt ccaggccggt tcccgtcgca agaagagaaa gtttcttgaa 840

accgtggaac tccagatcag cttgaagaac tacgaccctc agaaggacaa gcgtttctca 900

ggcactgtca ggctgaagac caccccgcgg cccaagttct ctgtctgcgt tcttggagac 960

cagcagcatt gtgatgaagc aaaggccgct gaactgccac acatggacat tgaggctctt 1020

aagaagctca ataagaacaa gaaactggtc aagaagctgg caaagaagta cgatgctttc 1080

ctggcctctg agtctctgat caagcagatt cctcgtatcc tgggccctgg cctcaacaag 1140

gcaggaaagt tccctctctt gctcacccat aatgagaacc tgggcaccaa ggtggatgag 1200

gtgaaatcca ccatcaaatt ccagatgaag aaggttctgt gtctggctgt ggccgtgggt 1260

catgtgaaga tgagtgagga agagctggtc tacaacatcc acctggctgt caacttcctg 1320

gtgtctctgc tgaagaagaa ctggcaaaac gtcagagctc tttacatcaa gagcaccatg 1380

ggaaaaccac agcgcctcta ctag 1404

<210> 13
<211> 1040
<212> DNA
<213> Artificial Sequence

<220>
<223> Promotor Gata2 from Danio rerio

<400> 13
agtgtccgcg cttagaaaat gcttggaaaa gtttacttga cactacagac ccgaaagcac 60

cactatagtc cgttagatta ttaaaatatc tgtcgttagc ttaatgcttc aggtggcgat 120

gctctgttta ctttctgagc atgcttttat ttttgaagga gtgtagaggt cctgcttctt      180

tgcattcaag ggtggccagg gacacctgta agagaataac agatagaagc atctcagtgt      240

tgacgttaaa cacagtgcaa gacagatctt aattcacacg acgacgcctc agttaatata      300

agtaggctaa cttttgtagt caatcaatta aatcgctcaa ttaaatgctc gcaatttcaa      360

ataactatcg ggaagtactc acggcgcaaa gttgcatttc agcactccaa tcctgcgttt      420

atttttcccg tgttagtcct gcgcaaagaa tgttaatcct caagatgcct gttgacgttc      480

gtaattcgcc actggtttaa attgtccaca gcccgattgt taaagtctcc ctaaaactct      540

gcgtctgtct aaagcggaga gaagtctcat taatgaattg tgcctgtgta ctgtcggaac      600

tggcgccagt agattccata tcttacgaac atgggcgggg acacgagctg gcagtgagcg      660

ccctgggaac caatagcaga gcgcgctgac tgccattggc tcggacagca tgctttccga      720

aatgccacgg cgactgtcgg cctactgctc cacatccaga gagctgagag actcatcatt      780

cagcgcagca atttattgag atctttttac cctttttaga gcttcacgtt gatggtgtgt      840

aatgatgtct ttttactcaa gtgctaacag aaaagcctaa aatctaagtt gcagcctaat      900

gctatttttt aaatttattt ttaggacatt aaatgatgtg aataatataa ttatagactt      960

gcaggattat atttaatttt taattgtgca gaaatgtatt aaaatgcctc tattctatta     1020

atgaatatcg ccgcaagctt                                                 1040

<210>  14
<211>  3809
<212>  DNA
<213>  Artificial Sequence

<220>
<223>  Genetic cassette Tol2_trap:TRAP


<220>
<221>  protein_bind
<222>  (1)..(148)
<223>  First Tol2 transposase recognition sequence

<220>
<221>  promoter
<222>  (564)..(1603)
<223>  Gata2p promoter (Danio rerio)

<220>
<221>  misc_feature
<222>  (1624)..(3027)
<223>  eGFLP-rpl10 fussion gene

<220>
<221>  polyA_signal
<222>  (3028)..(3233)

<220>
<221>  protein_bind

<222>   (3611)..(3809)
<223>   Second Tol2 transposase recognition sequence

<400>   14
cagaggtgta aaaagtactc aaaaatttta ctcaagtgaa agtacaagta cttagggaaa      60

attttactca attaaaagta aaagtatctg gctagaatct tacttgagta aaagtaaaaa     120

agtactccat taaaattgta cttgagtatt aaggaagtaa aagtaaaagc aagaaagaaa     180

actagagatt cttgtttaag cttttaatct caaaaaacat taaatgaaat gcatacaagg     240

ttttatcctg ctttagaact gtttgtattt aattatcaaa ctataagaca gacaatctaa     300

tgccagtaca cgctactcaa agttgtaaaa cctcagattt aacttcagta gaagctgatt     360

ctcaaaattg ttagtgtcaa gcctagctct tttggggctg aaaagcaatc ctgcagtgct     420

gaaaagcctc tcacaggcag ccgatgcggg aagaggtgta ttagtcttga tagagaggct     480

gcaaatagca ggaaacgtga gcagagactc cctggtgtct gaaacacagg ccagatgggc     540

cctcgagtgc tagcatgtcg acaagtgtcc gcgcttagaa aatgcttgga aaagtttact     600

tgacactaca gacccgaaag caccactata gtccgttaga ttattaaaat atctgtcgtt     660

agcttaatgc ttcaggtggc gatgctctgt ttactttctg agcatgcttt tatttttgaa     720

ggagtgtaga ggtcctgctt ctttgcattc aagggtggcc agggacacct gtaagagaat     780

aacagataga agcatctcag tgttgacgtt aaacacagtg caagacagat cttaattcac     840

acgacgacgc ctcagttaat ataagtaggc taacttttgt agtcaatcaa ttaaatcgct     900

caattaaatg ctcgcaattt caaataacta tcgggaagta ctcacggcgc aaagttgcat     960

ttcagcactc caatcctgcg tttatttttc ccgtgttagt cctgcgcaaa gaatgttaat    1020

cctcaagatg cctgttgacg ttcgtaattc gccactggtt taaattgtcc acagcccgat    1080

tgttaaagtc tccctaaaac tctgcgtctg tctaaagcgg agagaagtct cattaatgaa    1140

ttgtgcctgt gtactgtcgg aactggcgcc agtagattcc atatcttacg aacatggcgg    1200

gggacacgag ctggcagtga gcgccctggg aaccaatagc agagcgcgct gactgccatt    1260

ggctcggaca gcatgctttc cgaaatgcca cggcgactgt cggcctactg ctccacatcc    1320

agagagctga gagactcatc attcagcgca gcaatttatt gagatctttt tacccttttt     1380

agagcttcac gttgatggtg tgtaatgatg tctttttact caagtgctaa cagaaaagcc    1440

taaaatctaa gttgcagcct aatgctattt tttaaattta tttttaggac attaaatgat    1500

gtgaataata taattataga cttgcaggat tatatttaat ttttaattgt gcagaaatgt    1560

attaaaatgc ctctattcta ttaatgaata tcgccgcaag cttgggatcc accggtcgcc    1620

accatggtga gcaagggcga ggagctgttc accggggtgg tgcccatcct ggtcgagctg    1680

gacggcgacg taaacggcca caagttcagc gtgtccggcg agggcgaggg cgatgccacc    1740

tacggcaagc tgaccctgaa gttcatctgc accaccggca agctgcccgt gccctggccc    1800

accctcgtga ccaccctgac ctacggcgtg cagtgcttca gccgctaccc cgaccacatg 1860

aagcagcacg acttcttcaa gtccgccatg cccgaaggct acgtccagga gcgcaccatc 1920

ttcttcaagg acgacggcaa ctacaagacc cgcgccgagg tgaagttcga gggcgacacc 1980

ctggtgaacc gcatcgagct gaagggcatc gacttcaagg aggacggcaa catcctgggg 2040

cacaagctgg agtacaacta caacagccac aacgtctata tcatggccga caagcagaag 2100

aacggcatca aggtgaactt caagatccgc cacaacatcg aggacggcag cgtgcagctc 2160

gccgaccact accagcagaa cacccccatc ggcgacggcc ccgtgctgct gcccgacaac 2220

cactacctga gcacccagtc cgccctgagc aaagacccca acgagaagcg cgatcacatg 2280

gtcctgctgg agttcgtgac cgccgccggg atcactctcg gcatggacga gctgtacaag 2340

tccggccgga ctcagatctc gagctcaagc ttcgaattca gcaaggtctc gagagacacg 2400

ttgtacgagg ccgttaagga ggtccaggcc ggttcccgtc gcaagaagag aaagtttctt 2460

gaaaccgtgg aactccagat cagcttgaag aactacgacc ctcagaagga caagcgtttc 2520

tcaggcactg tcaggctgaa gaccaccccg cggcccaagt tctctgtctg cgttcttgga 2580

gaccagcagc attgtgatga agcaaaggcc gctgaactgc cacacatgga cattgaggct 2640

cttaagaagc tcaataagaa caagaaactg gtcaagaagc tggcaaagaa gtacgatgct 2700

ttcctggcct ctgagtctct gatcaagcag attcctcgta tcctgggccc tggcctcaac 2760

aaggcaggaa agtttccctc tctgctcacc cataatgaga acctgggcac caaggtggat 2820

gaggtgaaat ccaccatcaa attccagatg aagaaggttc tgtgtctggc tgtggccgtg 2880

ggtcatgtga agatgagtga ggaagagctg gtctacaaca tccacctggc tgtcaacttc 2940

ctggtgtctc tgctgaagaa gaactggcaa aacgtcagag ctctttacat caagagcacc 3000

atgggaaaac cacagcgcct ctactaggat cgatgatgat ccagacatga taagatacat 3060

tgatgagttt ggacaaacca caactagaat gcagtgaaaa aaatgcttta tttgtgaaat 3120

ttgtgatgct attgctttat ttgtaaccat tataagctgc aataaacaag ttaacaacaa 3180

caattgcatt cattttatgt ttcaggttca gggggaggtg tgggaggttt tttaaagcaa 3240

gtaaaacctc tacaaatgtg gtatggctga ttatgatcct ctagatcaga tctgcgaaga 3300

tacggccacg ggtgctcttg atcctgtggc tgattttgga ctgtgctgct cgcagctgct 3360

gatgaatcac atacttcctc cattttcttc cactgattga ctgttataat ttccctaatt 3420

tccaggtcaa ggtgctgtgc attgtggtaa tagatgtgac atgacgtcac ttccaaagga 3480

ccaatgaaca tgtctgacca atttcatata atgtgaaaac gattttcata ggcagaataa 3540

ataacattta aattaaactg ggcatcagcg caattcaatt ggtttggtaa tagcaaggga 3600

aaatagaatg aagtgatctc caaaaaataa gtactttttg actgtaaata aaattgtaag 3660

gagtaaaaag tacttttttt tctaaaaaaa tgtaattaag taaaagtaaa agtattgatt 3720

tttaattgta ctcaagtaaa gtaaaaatcc ccaaaaataa tacttaagta cagtaatcaa 3780

gtaaaattac tcaagtactt tacacctct 3809

<210> 15
<211> 34
<212> DNA
<213> Artificial Sequence

<220>
<223> LoxP recognition sites

<400> 15
ataacttcgt ataatgtatg ctatacgaag ttat 34

<210> 16
<211> 52
<212> DNA
<213> Artificial Sequence

<220>
<223> AttP landing site for integrase specific site

<400> 16
atgtggtcct ttagatccac tgacgtgggt cagtgtctct aaaggactcg cg 52

<210> 17
<211> 3929
<212> DNA
<213> Artificial Sequence

<220>
<223> Genetic cassette Tol2_trap:TRAP_LS

<220>
<221> protein_bind
<222> (1)..(148)
<223> First Tol2 transposase recognition sequence

<220>
<221> misc_recomb
<222> (328)..(361)
<223> LoxP site

<220>
<221> promoter
<222> (598)..(1637)
<223> Gata2 from Danio rerio

<220>
<221> misc_feature
<222> (1658)..(3061)
<223> eGFLP-rpl10a fusion gene

<220>
<221> polyA_signal
<222> (3076)..(3267)

<220>
<221> misc_recomb
<222> (3289)..(3322)
<223> LoxP site

<220>
<221> misc_feature
<222> (3326)..(3377)
<223> attP integrase site

<220>
<221> protein_bind
<222> (3731)..(3929)
<223> Second Tol2 transposase recognition sequence

<400> 17
cagaggtgta aaaagtactc aaaaatttta ctcaagtgaa agtacaagta cttagggaaa 60

attttactca attaaaagta aaagtatctg gctagaatct tacttgagta aaagtaaaaa 120

agtactccat taaaattgta cttgagtatt aaggaagtaa aagtaaaagc aagaaagaaa 180

actagagatt cttgtttaag cttttaatct caaaaaacat taaatgaaat gcatacaagg 240

ttttatcctg ctttagaact gtttgtattt aattatcaaa ctataagaca gacaatctaa 300

tgccagtaca cgctactcaa agttgtaata acttcgtata atgtatgcta tacgaagtta 360

taaacctcag atttaacttc agtagaagct gattctcaaa attgttagtg tcaagcctag 420

ctcttttggg gctgaaaagc aatcctgcag tgctgaaaag cctctcacag gcagccgatg 480

cgggaagagg tgtattagtc ttgatagaga ggctgcaaat agcaggaaac gtgagcagag 540

actccctggt gtctgaaaca caggccagat gggccctcga gtgctagcat gtcgacaagt 600

gtccgcgctt agaaaatgct tggaaaagtt tacttgacac tacagacccg aaagcaccac 660

tatagtccgt tagattatta aaatatctgt cgttagctta atgcttcagg tggcgatgct 720

ctgtttactt tctgagcatg cttttatttt tgaaggagtg tagaggtcct gcttctttgc 780

attcaagggt ggccagggac acctgtaaga gaataacaga tagaagcatc tcagtgttga 840

cgttaaacac agtgcaagac agatcttaat tcacacgacg acgcctcagt taatataagt 900

aggctaactt ttgtagtcaa tcaattaaat cgctcaatta aatgctcgca atttcaaata 960

actatcggga agtactcacg gcgcaaagtt gcatttcagc actccaatcc tgcgtttatt 1020

tttcccgtgt tagtcctgcg caaagaatgt taatcctcaa gatgcctgtt gacgttcgta 1080

attcgccact ggtttaaatt gtccacagcc cgattgttaa agtctcccta aaactctgcg 1140

tctgtctaaa gcggagagaa gtctcattaa tgaattgtgc ctgtgtactg tcggaactgg 1200

cgccagtaga ttccatatct tacgaacatg ggcggggaca cgagctggca gtgagcgccc 1260

tgggaaccaa tagcagagcg cgctgactgc cattggctcg gacagcatgc tttccgaaat 1320

gccacggcga ctgtcggcct actgctccac atccagagag ctgagagact catcattcag 1380

cgcagcaatt tattgagatc tttttaccct ttttagagct tcacgttgat ggtgtgtaat 1440

gatgtctttt tactcaagtg ctaacagaaa agcctaaaat ctaagttgca gcctaatgct 1500

attttttaaa tttattttta ggacattaaa tgatgtgaat aatataatta tagacttgca 1560

ggattatatt taatttttaa ttgtgcagaa atgtattaaa atgcctctat tctattaatg 1620

aatatcgccg caagcttggg atccaccggt cgccaccatg gtgagcaagg gcgaggagct 1680

gttcaccggg gtggtgccca tcctggtcga gctggacggc gacgtaaacg gccacaagtt 1740

cagcgtgtcc ggcgagggcg agggcgatgc cacctacggc aagctgaccc tgaagttcat 1800

ctgcaccacc ggcaagctgc ccgtgccctg gcccaccctc gtgaccaccc tgacctacgg 1860

cgtgcagtgc ttcagccgct accccgacca catgaagcag cacgacttct tcaagtccgc 1920

catgcccgaa ggctacgtcc aggagcgcac catcttcttc aaggacgacg gcaactacaa 1980

gacccgcgcc gaggtgaagt tcgagggcga caccctggtg aaccgcatcg agctgaaggg 2040

catcgacttc aaggaggacg gcaacatcct ggggcacaag ctggagtaca actacaacag 2100

ccacaacgtc tatatcatgg ccgacaagca gaagaacggc atcaaggtga acttcaagat 2160

ccgccacaac atcgaggacg gcagcgtgca gctcgccgac cactaccagc agaacacccc 2220

catcggcgac ggccccgtgc tgctgcccga caaccactac ctgagcaccc agtccgccct 2280

gagcaaagac cccaacgaga agcgcgatca catggtcctg ctggagttcg tgaccgccgc 2340

cgggatcact ctcggcatgg acgagctgta caagtccggc cggactcaga tctcgagctc 2400

aagcttcgaa ttcagcaagg tctcgagaga cacgttgtac gaggccgtta aggaggtcca 2460

ggccggttcc cgtcgcaaga agagaaagtt tcttgaaacc gtggaactcc agatcagctt 2520

gaagaactac gaccctcaga aggacaagcg tttctcaggc actgtcaggc tgaagaccac 2580

cccgcggccc aagttctctg tctgcgttct tggagaccag cagcattgtg atgaagcaaa 2640

ggccgctgaa ctgccacaca tggacattga ggctcttaag aagctcaata agaacaagaa 2700

actggtcaag aagctggcaa agaagtacga tgctttcctg gcctctgagt ctctgatcaa 2760

gcagattcct cgtatcctgg gccctggcct caacaaggca ggaaagtttc cctctctgct 2820

cacccataat gagaacctgg gcaccaaggt ggatgaggtg aaatccacca tcaaattcca 2880

gatgaagaag gttctgtgtc tggctgtggc cgtgggtcat gtgaagatga gtgaggaaga 2940

gctggtctac aacatccacc tggctgtcaa cttcctggtg tctctgctga agaagaactg 3000

gcaaaacgtc agagctcttt acatcaagag caccatggga aaaccacagc gcctctacta 3060

ggatcgatga tgatccagac atgataagat acattgatga gtttggacaa accacaacta 3120

gaatgcagtg aaaaaaatgc tttatttgtg aaatttgtga tgctattgct ttatttgtaa 3180

ccattataag ctgcaataaa caagttaaca acaacaattg cattcatttt atgtttcagg 3240

ttcaggggga ggtgtgggag gttttttaaa gcaagtaaaa cctctacaat aacttcgtat 3300

aatgtatgct atacgaagtt ataatatgtg gtcctttaga tccactgacg tgggtcagtg 3360

tctctaaagg actcgcggtg gtatggctga ttatgatcct ctagatcaga tctgcgaaga 3420

tacggccacg ggtgctcttg atcctgtggc tgattttgga ctgtgctgct cgcagctgct 3480

gatgaatcac atacttcctc cattttcttc cactgattga ctgttataat ttccctaatt 3540

tccaggtcaa ggtgctgtgc attgtggtaa tagatgtgac atgacgtcac ttccaaagga 3600

ccaatgaaca tgtctgacca atttcatata atgtgaaaac gattttcata ggcagaataa 3660

ataacattta aattaaactg ggcatcagcg caattcaatt ggtttggtaa tagcaaggga 3720

aaatagaatg aagtgatctc caaaaaataa gtactttttg actgtaaata aaattgtaag 3780

gagtaaaaag tacttttttt tctaaaaaaa tgtaattaag taaaagtaaa agtattgatt 3840

tttaattgta ctcaagtaaa gtaaaaatcc ccaaaaataa tacttaagta cagtaatcaa 3900

gtaaaattac tcaagtactt tacacctct 3929

<210> 18
<211> 8614
<212> DNA
<213> Artificial Sequence

<220>
<223> Tol2_trap:TRAP Vector

<220>
<221> polyA_signal
<222> (14)..(205)

<220>
<221> protein_bind
<222> (583)..(781)
<223> First Tol2 transposase recognition sequence

<220>
<221> rep_origin
<222> (1292)..(1920)
<223> ColE1 origin

<220>
<221> misc_feature
<222> (2072)..(2731)
<223> Ampicilin resistance

<220>
<221> rep_origin
<222> (3070)..(3510)
<223> F1 ori

<220>
<221> protein_bind
<222> (5587)..(5734)
<223> Second Tol2 transposase recognition sequence

<220>

<221>  promoter
<222>  (6150)..(7189)
<223>  Promotor Gata2 from Danio rerio

<220>
<221>  misc_feature
<222>  (7210)..(8613)
<223>  Fusion gene eGFP+rpL10a

<400>  18
atcgatgatg atccagacat gataagatac attgatgagt ttggacaaac cacaactaga      60

atgcagtgaa aaaaatgctt tatttgtgaa atttgtgatg ctattgcttt atttgtaacc     120

attataagct gcaataaaca agttaacaac aacaattgca ttcattttat gtttcaggtt     180

cagggggagg tgtgggaggt tttttaaagc aagtaaaacc tctacaaatg tggtatggct     240

gattatgatc ctctagatca gatctgcgaa gatacggcca cgggtgctct tgatcctgtg     300

gctgattttg gactgtgctg ctcgcagctg ctgatgaatc acatacttcc tccattttct     360

tccactgatt gactgttata atttccctaa tttccaggtc aaggtgctgt gcattgtggt     420

aatagatgtg acatgacgtc acttccaaag gaccaatgaa catgtctgac caatttcata     480

taatgtgaaa acgattttca taggcagaat aaataacatt taaattaaac tgggcatcag     540

cgcaattcaa ttggtttggt aatagcaagg gaaaatagaa tgaagtgatc tccaaaaaat     600

aagtactttt tgactgtaaa taaaattgta aggagtaaaa agtacttttt tttctaaaaa     660

aatgtaatta agtaaaagta aaagtattga tttttaattg tactcaagta aagtaaaaat     720

ccccaaaaat aatacttaag tacagtaatc aagtaaaatt actcaagtac tttacacctc     780

tggttcttga ccccctacct tcagcaagcc cagcagatcc actagttcta gagcggccgc     840

caccgcggtg gagctccagc ttttgttccc tttagtgagg gttaattgcg cgcttggcgt     900

aatcatggtc atagctgttt cctgtgtgaa attgttatcc gctcacaatt ccacacaaca     960

tacgagccgg aagcataaag tgtaaagcct ggggtgccta atgagtgagc taactcacat    1020

taattgcgtt gcgctcactg cccgctttcc agtcgggaaa cctgtcgtgc cagctgcatt    1080

aatgaatcgg ccaacgcgcg gggagaggcg gtttgcgtat tgggcgctct tccgcttcct    1140

cgctcactga ctcgctgcgc tcggtcgttc ggctgcggcg agcggtatca gctcactcaa    1200

aggcggtaat acggttatcc acagaatcag gggataacgc aggaaagaac atgtgagcaa    1260

aaggccagca aaaggccagg aaccgtaaaa aggccgcgtt gctggcgttt ttccataggc    1320

tccgcccccc tgacgagcat cacaaaaatc gacgctcaag tcagaggtgg cgaaacccga    1380

caggactata aagataccag gcgtttcccc ctggaagctc cctcgtgcgc tctcctgttc    1440

cgaccctgcc gcttaccgga tacctgtccg cctttctccc ttcgggaagc gtggcgcttt    1500

ctcatagctc acgctgtagg tatctcagtt cggtgtaggt cgttcgctcc aagctgggct    1560

gtgtgcacga accccccgtt cagcccgacc gctgcgcctt atccggtaac tatcgtcttg    1620

agtccaaccc ggtaagacac gacttatcgc cactggcagc agccactggt aacaggatta 1680

gcagagcgag gtatgtaggc ggtgctacag agttcttgaa gtggtggcct aactacggct 1740

acactagaag gacagtattt ggtatctgcg ctctgctgaa gccagttacc ttcggaaaaa 1800

gagttggtag ctcttgatcc ggcaaacaaa ccaccgctgg tagcggtggt ttttttgttt 1860

gcaagcagca gattacgcgc agaaaaaaag gatctcaaga agatcctttg atcttttcta 1920

cggggtctga cgctcagtgg aacgaaaact cacgttaagg gattttggtc atgagattat 1980

caaaaaggat cttcacctag atccttttaa attaaaaatg aagttttaaa tcaatctaaa 2040

gtatatatga gtaaacttgg tctgacagtt accaatgctt aatcagtgag gcacctatct 2100

cagcgatctg tctatttcgt tcatccatag ttgcctgact ccccgtcgtg tagataacta 2160

cgatacggga gggcttacca tctggcccca gtgctgcaat gataccgcga gacccacgct 2220

caccggctcc agatttatca gcaataaacc agccagccgg aagggccgag cgcagaagtg 2280

gtcctgcaac tttatccgcc tccatccagt ctattaattg ttgccgggaa gctagagtaa 2340

gtagttcgcc agttaatagt ttgcgcaacg ttgttgccat tgctacaggc atcgtggtgt 2400

cacgctcgtc gtttggtatg gcttcattca gctccggttc ccaacgatca aggcgagtta 2460

catgatcccc catgttgtgc aaaaaagcgg ttagctcctt cggtcctccg atcgttgtca 2520

gaagtaagtt ggccgcagtg ttatcactca tggttatggc agcactgcat aattctctta 2580

ctgtcatgcc atccgtaaga tgcttttctg tgactggtga gtactcaacc aagtcattct 2640

gagaatagtg tatgcggcga ccgagttgct cttgcccggc gtcaatacgg gataataccg 2700

cgccacatag cagaacttta aaagtgctca tcattggaaa acgttcttcg gggcgaaaac 2760

tctcaaggat cttaccgctg ttgagatcca gttcgatgta acccactcgt gcacccaact 2820

gatcttcagc atcttttact ttcaccagcg tttctgggtg agcaaaaaca ggaaggcaaa 2880

atgccgcaaa aaagggaata agggcgacac ggaaatgttg aatactcata ctcttccttt 2940

ttcaatatta ttgaagcatt tatcagggtt attgtctcat gagcggatac atatttgaat 3000

gtatttagaa aaataaacaa ataggggttc cgcgcacatt tccccgaaaa gtgccaccta 3060

aattgtaagc gttaatattt tgttaaaatt cgcgttaaat ttttgttaaa tcagctcatt 3120

ttttaaccaa taggccgaaa tcggcaaaat cccttataaa tcaaaagaat agaccgagat 3180

agggttgagt gttgttccag tttggaacaa gagtccacta ttaaagaacg tggactccaa 3240

cgtcaaaggg cgaaaaaccg tctatcaggg cgatggccca ctacgtgaac catcacccta 3300

atcaagtttt ttggggtcga ggtgccgtaa agcactaaat cggaacccta aagggagccc 3360

ccgatttaga gcttgacggg gaaagccggc gaacgtggcg agaaaggaag ggaagaaagc 3420

gaaaggagcg ggcgctaggg cgctggcaag tgtagcggtc acgctgcgcg taaccaccac 3480

acccgccgcg cttaatgcgc cgctacaggg cgcgtcccat tcgccattca ggctgcgcaa 3540

ctgttgggaa gggcgatcgg tgcgggcctc ttcgctatta cgccagctgg cgaaaggggg 3600

atgtgctgca aggcgattaa gttgggtaac gccagggttt tcccagtcac gacgttgtaa 3660

aacgacggcc agtgagcgcg cgtaatacga ctcactatag ggcgaattgg gtaccaaata 3720

gtaggaatta cccacctgta caagtgctga aaacttggat gaataagccc gtttgccatt 3780

ttctactgct attttaaatc ttttctgtat ttgtctgcat ttgtctttac ccttgaataa 3840

atgttttagt tgtttttttt caattatgac tgtgtttaac agacattatt acaataatat 3900

gtaatagtag tacacactat tattatgtaa tagtacttgt tgactgtatt tgagaactgg 3960

accgagtgag tgttacgtca cccattcaaa atgacttact tctggctcca acaaaatgaa 4020

gttaattcag ttgccatttt tcactgtatg gacatcgccg tgttggagct agacgtcgcc 4080

aataagcaag aaagagccga gatgcgtcga gtctgagtca ccgttcctat ggcaacccct 4140

ctaaccaatc agaagtaagc ttgttggaag tccacagcct accacttgaa agcgggctgc 4200

acaaaatctg tcaaacgttt tgaacgttgg atgtgagagc acatactttt attaaggcat 4260

ctggttggtc agtttataat atcaacaact tgggctacag aaaagaaaag ttattacaga 4320

aattatgatt aacaagtaca tgttaaataa agattttaat atgaatgcca ccactggagc 4380

attcatgcca tttggagctt cttcctgttt ggatcactag aaggaggagg tcactcatta 4440

cagttctcat atacagtcgt tggttggttg gttggttggt tggttggtag attgattgat 4500

tgattgattg attgattgat tgattgattg attgattgat tgattgattg attgattgat 4560

tgattgattg attgattgat tgattgattg attggtagtc aaaataagaa ataatttcca 4620

cagattcatt acagaaatga ttaaatgcat acataaaaaa ctgggggggg ggggatacaa 4680

caacacactt aagtcacatt tgcctacgta aagaaaagta aagaaaatca atagctatat 4740

tttacatctc ctttttttgc tgtcttttaa ttagccttgt tttgctgtta tcttgattga 4800

aactgtaact tcttcacctg cttcttttct ttgtaggttt tgccagccca gaaacaggca 4860

tggctgtgca aggccagagc accatgcaca atgcgctgca tgtcttcatg aacggctcaa 4920

tgtcctcagt ccagggctca gccaacgacc ccatcttcct ccttcaccat gctttcattg 4980

acaggtaaca aacacgtcat gacattagac tgcacagttt ttgacaaagt tcatacaatc 5040

tgttgtttat agctgctaca attagtgaag tttgtgaatg tacttggatg agcagcgaaa 5100

gatcaattga gatcaattgt tagagtttgg ttgccctgca gagcaaagaa caaaaaataa 5160

tctggtggct ttactgcgtg aggttattat tggtggaata gaaacacaaa acataattgc 5220

atttatttgt ttaatttttt atcttatctt aactttcatc ttgcatattt gtttcttaca 5280

tcatttctag catctttgag cgctggctaa gaactcatca gcctccccgg tccatctacc 5340

cacgtaccaa tgcaccaatt ggccacaatg acggctacta catggtgcca ttccttcctc 5400

tttataggaa tggagactac ctcctgtcca acaaggctct tggatacgag tacgcctacc 5460

tgttggaccc aggtcattgc acaacaccag aaatgccctc tgatctgcaa aagacgtgaa 5520

tatctgttca gacacccata tccactctgt tccacacagg tcagaggttt gtccaggagt 5580

tcttgacaga ggtgtaaaaa gtactcaaaa attttactca agtgaaagta caagtactta 5640

gggaaaattt tactcaatta aaagtaaaag tatctggcta gaatcttact tgagtaaaag 5700

taaaaaagta ctccattaaa attgtacttg agtattaagg aagtaaaagt aaaagcaaga 5760

aagaaaacta gagattcttg tttaagcttt taatctcaaa aaacattaaa tgaaatgcat 5820

acaaggtttt atcctgcttt agaactgttt gtatttaatt atcaaactat aagacagaca 5880

atctaatgcc agtacacgct actcaaagtt gtaaaacctc agatttaact tcagtagaag 5940

ctgattctca aaattgttag tgtcaagcct agctcttttg gggctgaaaa gcaatcctgc 6000

agtgctgaaa agcctctcac aggcagccga tgcgggaaga ggtgtattag tcttgataga 6060

gaggctgcaa atagcaggaa acgtgagcag agactccctg gtgtctgaaa cacaggccag 6120

atgggccctc gagtgctagc atgtcgacaa gtgtccgcgc ttagaaaatg cttggaaaag 6180

tttacttgac actacagacc cgaaagcacc actatagtcc gttagattat taaaatatct 6240

gtcgttagct taatgcttca ggtggcgatg ctctgtttac tttctgagca tgcttttatt 6300

tttgaaggag tgtagaggtc ctgcttcttt gcattcaagg gtggccaggg acacctgtaa 6360

gagaataaca gatagaagca tctcagtgtt gacgttaaac acagtgcaag acagatctta 6420

attcacacga cgacgcctca gttaatataa gtaggctaac ttttgtagtc aatcaattaa 6480

atcgctcaat taaatgctcg caatttcaaa taactatcgg gaagtactca cggcgcaaag 6540

ttgcatttca gcactccaat cctgcgttta tttttcccgt gttagtcctg cgcaaagaat 6600

gttaatcctc aagatgcctg ttgacgttcg taattcgcca ctggtttaaa ttgtccacag 6660

cccgattgtt aaagtctccc taaaactctg cgtctgtcta aagcggagag aagtctcatt 6720

aatgaattgt gcctgtgtac tgtcggaact ggcgccagta gattccatat cttacgaaca 6780

tgggcgggga cacgagctgg cagtgagcgc cctgggaacc aatagcagag cgcgctgact 6840

gccattggct cggacagcat gctttccgaa atgccacggc gactgtcggc ctactgctcc 6900

acatccagag agctgagaga ctcatcattc agcgcagcaa tttattgaga tctttttacc 6960

ctttttagag cttcacgttg atggtgtgta atgatgtctt tttactcaag tgctaacaga 7020

aaagcctaaa atctaagttg cagcctaatg ctatttttta aatttatttt taggacatta 7080

aatgatgtga ataatataat tatagacttg caggattata tttaattttt aattgtgcag 7140

aaatgtatta aaatgcctct attctattaa tgaatatcgc cgcaagcttg ggatccaccg 7200

gtcgccacca tggtgagcaa gggcgaggag ctgttcaccg gggtggtgcc catcctggtc 7260

gagctggacg gcgacgtaaa cggccacaag ttcagcgtgt ccggcgaggg cgagggcgat 7320

gccacctacg gcaagctgac cctgaagttc atctgcacca ccggcaagct gcccgtgccc 7380

tggcccaccc tcgtgaccac cctgacctac ggcgtgcagt gcttcagccg ctaccccgac 7440

cacatgaagc agcacgactt cttcaagtcc gccatgcccg aaggctacgt ccaggagcgc 7500

accatcttct tcaaggacga cggcaactac aagacccgcg ccgaggtgaa gttcgagggc 7560

gacaccctgg tgaaccgcat cgagctgaag ggcatcgact tcaaggagga cggcaacatc 7620

ctggggcaca agctggagta caactacaac agccacaacg tctatatcat ggccgacaag 7680

cagaagaacg gcatcaaggt gaacttcaag atccgccaca acatcgagga cggcagcgtg 7740

cagctcgccg accactacca gcagaacacc cccatcggcg acggccccgt gctgctgccc 7800

gacaaccact acctgagcac ccagtccgcc ctgagcaaag accccaacga gaagcgcgat 7860

cacatggtcc tgctggagtt cgtgaccgcc gccgggatca ctctcggcat ggacgagctg 7920

tacaagtccg gccggactca gatctcgagc tcaagcttcg aattcagcaa ggtctcgaga 7980

gacacgttgt acgaggccgt taaggaggtc caggccggtt cccgtcgcaa gaagagaaag 8040

tttcttgaaa ccgtggaact ccagatcagc ttgaagaact acgaccctca gaaggacaag 8100

cgtttctcag gcactgtcag gctgaagacc accccgcggc ccaagttctc tgtctgcgtt 8160

cttggagacc agcagcattg tgatgaagca aaggccgctg aactgccaca catggacatt 8220

gaggctctta agaagctcaa taagaacaag aaactggtca agaagctggc aaagaagtac 8280

gatgctttcc tggcctctga gtctctgatc aagcagattc ctcgtatcct gggccctggc 8340

ctcaacaagg caggaaagtt tccctctctg ctcacccata atgagaacct gggcaccaag 8400

gtggatgagg tgaaatccac catcaaattc cagatgaaga aggttctgtg tctggctgtg 8460

gccgtgggtc atgtgaagat gagtgaggaa gagctggtct acaacatcca cctggctgtc 8520

aacttcctgg tgtctctgct gaagaagaac tggcaaaacg tcagagctct ttacatcaag 8580

agcaccatgg gaaaaccaca gcgcctctac tagg 8614

<210> 19
<211> 8721
<212> DNA
<213> Artificial Sequence

<220>
<223> Vector Tol2_trap:TRAP_LS


<220>
<221> polyA_signal
<222> (14)..(205)

<220>
<221> misc_recomb
<222> (262)..(295)

<223>  LoxP site

<220>
<221>  misc_feature
<222>  (354)..(392)
<223>  attP integrase site

<220>
<221>  protein_bind
<222>  (656)..(854)
<223>  First Tol2 transposase recognition sequence

<220>
<221>  rep_origin
<222>  (1365)..(1993)
<223>  ColE1 origin

<220>
<221>  misc_feature
<222>  (2145)..(2804)
<223>  Ampicilin resistance

<220>
<221>  rep_origin
<222>  (3143)..(3583)
<223>  F1 ori

<220>
<221>  protein_bind
<222>  (5660)..(5807)
<223>  Second Tol2 transposase recognition sequence

<220>
<221>  misc_recomb
<222>  (6192)..(6225)
<223>  LoxP site

<220>
<221>  promoter
<222>  (6257)..(7296)
<223>  Promotor Gata2 from Danio rerio

<220>
<221>  misc_feature
<222>  (7317)..(8720)
<223>  Fusion gene eGFP-rpl10a

<400>  19
atcgatgatg atccagacat gataagatac attgatgagt ttggacaaac cacaactaga      60

atgcagtgaa aaaaatgctt tatttgtgaa atttgtgatg ctattgcttt atttgtaacc     120

attataagct gcaataaaca agttaacaac aacaattgca ttcattttat gtttcaggtt     180

cagggggagg tgtgggaggt tttttaaagc aagtaaaacc tctacaaatg tggtatggct     240

gattatgatc ctctagatca gataacttcg tataatgtat gctatacgaa gttatatctg     300

cgaagatacg gccacgggtg ctcttgatcc tgtggctgat tttggactgt gctcccccaa     360

ctgagagaac tcaaaggtta ccccagttgg gggctcgcag ctgctgatga atcacatact     420

tcctccattt tcttccactg attgactgtt ataatttccc taatttccag gtcaaggtgc     480

tgtgcattgt ggtaatagat gtgacatgac gtcacttcca aaggaccaat gaacatgtct 540

gaccaatttc atataatgtg aaaacgattt tcataggcag aataaataac atttaaatta 600

aactgggcat cagcgcaatt caattggttt ggtaatagca agggaaaata gaatgaagtg 660

atctccaaaa aataagtact ttttgactgt aaataaaatt gtaaggagta aaaagtactt 720

ttttttctaa aaaaatgtaa ttaagtaaaa gtaaaagtat tgatttttaa ttgtactcaa 780

gtaaagtaaa aatccccaaa aataatactt aagtacagta atcaagtaaa attactcaag 840

tactttacac ctctggttct tgacccccta ccttcagcaa gcccagcaga tccactagtt 900

ctagagcggc cgccaccgcg gtggagctcc agcttttgtt ccctttagtg agggttaatt 960

gcgcgcttgg cgtaatcatg gtcatagctg tttcctgtgt gaaattgtta tccgctcaca 1020

attccacaca acatacgagc cggaagcata aagtgtaaag cctggggtgc ctaatgagtg 1080

agctaactca cattaattgc gttgcgctca ctgcccgctt tccagtcggg aaacctgtcg 1140

tgccagctgc attaatgaat cggccaacgc gcggggagag gcggtttgcg tattgggcgc 1200

tcttccgctt cctcgctcac tgactcgctg cgctcggtcg ttcggctgcg gcgagcggta 1260

tcagctcact caaaggcggt aatacggtta tccacagaat caggggataa cgcaggaaag 1320

aacatgtgag caaaaggcca gcaaaaggcc aggaaccgta aaaaggccgc gttgctggcg 1380

tttttccata ggctccgccc ccctgacgag catcacaaaa atcgacgctc aagtcagagg 1440

tggcgaaacc cgacaggact ataaagatac caggcgtttc cccctggaag ctccctcgtg 1500

cgctctcctg ttccgaccct gccgcttacc ggatacctgt ccgcctttct cccttcggga 1560

agcgtggcgc tttctcatag ctcacgctgt aggtatctca gttcggtgta ggtcgttcgc 1620

tccaagctgg gctgtgtgca cgaacccccc gttcagcccg accgctgcgc cttatccggt 1680

aactatcgtc ttgagtccaa cccggtaaga cacgacttat cgccactggc agcagccact 1740

ggtaacagga ttagcagagc gaggtatgta ggcggtgcta cagagttctt gaagtggtgg 1800

cctaactacg gctacactag aaggacagta tttggtatct gcgctctgct gaagccagtt 1860

accttcggaa aaagagttgg tagctcttga tccggcaaac aaaccaccgc tggtagcggt 1920

ggtttttttg tttgcaagca gcagattacg cgcagaaaaa aaggatctca agaagatcct 1980

ttgatctttt ctacggggtc tgacgctcag tggaacgaaa actcacgtta agggattttg 2040

gtcatgagat tatcaaaaag gatcttcacc tagatccttt taaattaaaa atgaagtttt 2100

aaatcaatct aaagtatata tgagtaaact tggtctgaca gttaccaatg cttaatcagt 2160

gaggcaccta tctcagcgat ctgtctattt cgttcatcca tagttgcctg actccccgtc 2220

gtgtagataa ctacgatacg ggagggctta ccatctggcc ccagtgctgc aatgataccg 2280

cgagacccac gctcaccggc tccagattta tcagcaataa accagccagc cggaagggcc 2340

gagcgcagaa gtggtcctgc aactttatcc gcctccatcc agtctattaa ttgttgccgg 2400

gaagctagag taagtagttc gccagttaat agtttgcgca acgttgttgc cattgctaca 2460

ggcatcgtgg tgtcacgctc gtcgtttggt atggcttcat tcagctccgg ttcccaacga 2520

tcaaggcgag ttacatgatc ccccatgttg tgcaaaaaag cggttagctc cttcggtcct 2580

ccgatcgttg tcagaagtaa gttggccgca gtgttatcac tcatggttat ggcagcactg 2640

cataattctc ttactgtcat gccatccgta agatgctttt ctgtgactgg tgagtactca 2700

accaagtcat tctgagaata gtgtatgcgg cgaccgagtt gctcttgccc ggcgtcaata 2760

cgggataata ccgcgccaca tagcagaact ttaaaagtgc tcatcattgg aaaacgttct 2820

tcggggcgaa aactctcaag gatcttaccg ctgttgagat ccagttcgat gtaacccact 2880

cgtgcaccca actgatcttc agcatctttt actttcacca gcgtttctgg gtgagcaaaa 2940

acaggaaggc aaaatgccgc aaaaaaggga ataagggcga cacggaaatg ttgaatactc 3000

atactcttcc tttttcaata ttattgaagc atttatcagg gttattgtct catgagcgga 3060

tacatatttg aatgtattta gaaaaataaa caaatagggg ttccgcgcac atttccccga 3120

aaagtgccac ctaaattgta agcgttaata ttttgttaaa attcgcgtta aatttttgtt 3180

aaatcagctc attttttaac caataggccg aaatcggcaa aatcccttat aaatcaaaag 3240

aatagaccga gatagggttg agtgttgttc cagtttggaa caagagtcca ctattaaaga 3300

acgtggactc caacgtcaaa gggcgaaaaa ccgtctatca gggcgatggc ccactacgtg 3360

aaccatcacc ctaatcaagt tttttggggt cgaggtgccg taaagcacta aatcggaacc 3420

ctaaagggag cccccgattt agagcttgac ggggaaagcc ggcgaacgtg gcgagaaagg 3480

aagggaagaa agcgaaagga gcgggcgcta gggcgctggc aagtgtagcg gtcacgctgc 3540

gcgtaaccac cacacccgcc gcgcttaatg cgccgctaca gggcgcgtcc cattcgccat 3600

tcaggctgcg caactgttgg gaagggcgat cggtgcgggc ctcttcgcta ttacgccagc 3660

tggcgaaagg gggatgtgct gcaaggcgat taagttgggt aacgccaggg ttttcccagt 3720

cacgacgttg taaaacgacg gccagtgagc gcgcgtaata cgactcacta tagggcgaat 3780

tgggtaccaa atagtaggaa ttacccacct gtacaagtgc tgaaaacttg gatgaataag 3840

cccgtttgcc attttctact gctattttaa atcttttctg tatttgtctg catttgtctt 3900

tacccttgaa taaatgtttt agttgttttt tttcaattat gactgtgttt aacagacatt 3960

attacaataa tatgtaatag tagtacacac tattattatg taatagtact tgttgactgt 4020

atttgagaac tggaccgagt gagtgttacg tcacccattc aaaatgactt acttctggct 4080

ccaacaaaat gaagttaatt cagttgccat ttttcactgt atggacatcg ccgtgttgga 4140

gctagacgtc gccaataagc aagaaagagc cgagatgcgt cgagtctgag tcaccgttcc 4200

tatggcaacc cctctaacca atcagaagta agcttgttgg aagtccacag cctaccactt 4260

gaaagcgggc tgcacaaaat ctgtcaaacg ttttgaacgt tggatgtgag agcacatact 4320

tttattaagg catctggttg gtcagtttat aatatcaaca acttgggcta cagaaaagaa 4380

aagttattac agaaattatg attaacaagt acatgttaaa taaagatttt aatatgaatg 4440

ccaccactgg agcattcatg ccatttggag cttcttcctg tttggatcac tagaaggagg 4500

aggtcactca ttacagttct catatacagt cgttggttgg ttggttggtt ggttggttgg 4560

tagattgatt gattgattga ttgattgatt gattgattga ttgattgatt gattgattga 4620

ttgattgatt gattgattga ttgattgatt gattgattga ttgattggta gtcaaaataa 4680

gaaataattt ccacagattc attacagaaa tgattaaatg catacataaa aaactggggg 4740

gggggggata caacaacaca cttaagtcac atttgcctac gtaaagaaaa gtaaagaaaa 4800

tcaatagcta tattttacat ctcctttttt tgctgtcttt taattagcct tgttttgctg 4860

ttatcttgat tgaaactgta acttcttcac ctgcttcttt tctttgtagg ttttgccagc 4920

ccagaaacag gcatggctgt gcaaggccag agcaccatgc acaatgcgct gcatgtcttc 4980

atgaacggct caatgtcctc agtccagggc tcagccaacg accccatctt cctccttcac 5040

catgctttca ttgacaggta acaaacacgt catgacatta gactgcacag tttttgacaa 5100

agttcataca atctgttgtt tatagctgct acaattagtg aagtttgtga atgtacttgg 5160

atgagcagcg aaagatcaat tgagatcaat tgttagagtt tggttgccct gcagagcaaa 5220

gaacaaaaaa taatctggtg gctttactgc gtgaggttat tattggtgga atagaaacac 5280

aaaacataat tgcatttatt tgtttaattt tttatcttat cttaactttc atcttgcata 5340

tttgtttctt acatcatttc tagcatcttt gagcgctggc taagaactca tcagcctccc 5400

cggtccatct acccacgtac caatgcacca attggccaca atgacggcta ctacatggtg 5460

ccattccttc ctctttatag gaatggagac tacctcctgt ccaacaaggc tcttggatac 5520

gagtacgcct acctgttgga cccaggtcat tgcacaacac cagaaatgcc ctctgatctg 5580

caaaagacgt gaatatctgt tcagacaccc atatccactc tgttccacac aggtcagagg 5640

tttgtccagg agttcttgac agaggtgtaa aaagtactca aaaattttac tcaagtgaaa 5700

gtacaagtac ttagggaaaa ttttactcaa ttaaaagtaa aagtatctgg ctagaatctt 5760

acttgagtaa aagtaaaaaa gtactccatt aaaattgtac ttgagtatta aggaagtaaa 5820

agtaaaagca agaaagaaaa ctagagattc ttgtttaagc ttttaatctc aaaaaacatt 5880

aaatgaaatg catacaaggt tttatcctgc tttagaactg tttgtattta attatcaaac 5940

tataagacag acaatctaat gccagtacac gctactcaaa gttgtaaaac ctcagattta 6000

acttcagtag aagctgattc tcaaaattgt tagtgtcaag cctagctctt ttggggctga 6060

aaagcaatcc tgcagtgctg aaaagcctct cacaggcagc cgatgcggga agaggtgtat 6120

tagtcttgat agagaggctg caaatagcag gaaacgtgag cagagactcc ctggtgtctg 6180

aaacacaggc cataacttcg tataatgtat gctatacgaa gttatagatg ggccctcgag 6240

tgctagcatg tcgacaagtg tccgcgctta gaaaatgctt ggaaaagttt acttgacact 6300

acagacccga aagcaccact atagtccgtt agattattaa aatatctgtc gttagcttaa 6360

tgcttcaggt ggcgatgctc tgtttacttt ctgagcatgc ttttattttt gaaggagtgt 6420

agaggtcctg cttctttgca ttcaagggtg gccagggaca cctgtaagag aataacagat 6480

agaagcatct cagtgttgac gttaaacaca gtgcaagaca gatcttaatt cacacgacga 6540

cgcctcagtt aatataagta ggctaacttt tgtagtcaat caattaaatc gctcaattaa 6600

atgctcgcaa tttcaaataa ctatcgggaa gtactcacgg cgcaaagttg catttcagca 6660

ctccaatcct gcgtttattt ttcccgtgtt agtcctgcgc aaagaatgtt aatcctcaag 6720

atgcctgttg acgttcgtaa ttcgccactg gtttaaattg tccacagccc gattgttaaa 6780

gtctccctaa aactctgcgt ctgtctaaag cggagagaag tctcattaat gaattgtgcc 6840

tgtgtactgt cggaactggc gccagtagat tccatatctt acgaacatgg gcggggacac 6900

gagctggcag tgagcgccct gggaaccaat agcagagcgc gctgactgcc attggctcgg 6960

acagcatgct ttccgaaatg ccacggcgac tgtcggccta ctgctccaca tccagagagc 7020

tgagagactc atcattcagc gcagcaattt attgagatct tttacccttt tttagagctt 7080

cacgttgatg gtgtgtaatg atgtcttttt actcaagtgc taacagaaaa gcctaaaatc 7140

taagttgcag cctaatgcta ttttttaaat ttatttttag gacattaaat gatgtgaata 7200

atataattat agacttgcag gattatattt aatttttaat tgtgcagaaa tgtattaaaa 7260

tgcctctatt ctattaatga atatcgccgc aagcttggga tccaccggtc gccaccatgg 7320

tgagcaaggg cgaggagctg ttcaccgggg tggtgcccat cctggtcgag ctggacggcg 7380

acgtaaacgg ccacaagttc agcgtgtccg gcgagggcga gggcgatgcc acctacggca 7440

agctgaccct gaagttcatc tgcaccaccg gcaagctgcc cgtgccctgg cccaccctcg 7500

tgaccaccct gacctacggc gtgcagtgct tcagccgcta ccccgaccac atgaagcagc 7560

acgacttctt caagtccgcc atgcccgaag gctacgtcca ggagcgcacc atcttcttca 7620

aggacgacgg caactacaag acccgcgccg aggtgaagtt cgagggcgac accctggtga 7680

accgcatcga gctgaagggc atcgacttca aggaggacgg caacatcctg gggcacaagc 7740

tggagtacaa ctacaacagc cacaacgtct atatcatggc cgacaagcag aagaacggca 7800

tcaaggtgaa cttcaagatc cgccacaaca tcgaggacgg cagcgtgcag ctcgccgacc 7860

actaccagca gaacaccccc atcggcgacg gccccgtgct gctgcccgac aaccactacc 7920

tgagcaccca gtccgccctg agcaaagacc ccaacgagaa gcgcgatcac atggtcctgc 7980

tggagttcgt gaccgccgcc gggatcactc tcggcatgga cgagctgtac aagtccggcc 8040

ggactcagat ctcgagctca agcttcgaat tcagcaaggt ctcgagagac acgttgtacg 8100

aggccgttaa ggaggtccag gccggttccc gtcgcaagaa gagaaagttt cttgaaaccg 8160

tggaactcca gatcagcttg aagaactacg accctcagaa ggacaagcgt ttctcaggca 8220

ctgtcaggct gaagaccacc ccgcggccca agttctctgt ctgcgttctt ggagaccagc 8280

agcattgtga tgaagcaaag gccgctgaac tgccacacat ggacattgag gctcttaaga 8340

agctcaataa gaacaagaaa ctggtcaaga agctggcaaa gaagtacgat gctttcctgg 8400

cctctgagtc tctgatcaag cagattcctc gtatcctggg ccctggcctc aacaaggcag 8460

gaaagtttcc ctctctgctc acccataatg agaacctggg caccaaggtg gatgaggtga 8520

aatccaccat caaattccag atgaagaagg ttctgtgtct ggctgtggcc gtgggtcatg 8580

tgaagatgag tgaggaagag ctggtctaca acatccacct ggctgtcaac ttcctggtgt 8640

ctctgctgaa gaagaactgg caaaacgtca gagctcttta catcaagagc accatgggaa 8700

aaccacagcg cctctactag g 8721

<210> 20
<211> 2156
<212> DNA
<213> Oryzias latipes

<400> 20
acgtcatgtc acatctatta ccacaatgca cagcaccttg acctggaaat tagggaaatt 60

ataacagtca atcagtggaa gaaaatggag gaagtatgtg attcatcagc agctgcgagc 120

agcacagtcc aaaatcagcc acaggatcaa gagcacccgt ggccgtatct tcgcgaattc 180

ttttctttaa gtggtgtaaa taaagattca ttcaagatga aatgtgtcct ctgtctcccg 240

cttaataaag aaatatcggc cttcaaaagt tcgccatcaa acctaaggaa gcatattgag 300

agaatgcacc caaattacct caaaaactac tctaaattga cagcacagaa gagaaagatc 360

gggacctcca cccatgcttc cagcagtaag caactgaaag ttgactcagt tttcccagtc 420

aaacatgtgt ctccagtcac tgtgaacaaa gctatattaa ggtacatcat tcaaggactt 480

catcctttca gcactgttga tctgccatca tttaaagagc tgattagtac actgcagcct 540

ggcatttctg tcattacaag gcctacttta cgctccaaga tagctgaagc tgctctgatc 600

atgaaacaga aagtgactgc tgccatgagt gaagttgaat ggattgcaac cacaacggat 660

tgttggactg cacgtagaaa gtcattcatt ggtgtaactg ctcactggat caaccctgga 720

agtcttgaaa gacattccgc tgcacttgcc tgcaaaagat taatgggctc tcatactttt 780

gaggtactgg ccagtgccat gaatgatatc cactcagagt atgaaatacg tgacaaggtt 840

gtttgcacaa ccacagacag tggttccaac tttatgaagg ctttcagagt ttttggtgtg 900

gaaaacaatg atatcgagac tgaggcaaga aggtgtgaaa gtgatgacac tgattctgaa 960

ggctgtggtg agggaagtga tggtgtggaa ttccaagatg cctcacgagt cctggaccaa 1020

gacgatggct tcgaattcca gctaccaaaa catcaaaagt gtgcctgtca cttacttaac 1080

ctagtctcaa gcgttgatgc ccaaaaagct ctctcaaatg aacactacaa gaaactctac 1140

agatctgtct ttggcaaatg ccaagcttta tggaataaaa gcagccgatc ggctctagca 1200

gctgaagctg ttgaatcaga aagccggctt cagcttttaa ggccaaacca aacgcggtgg 1260

aattcaactt ttatggctgt tgacagaatt cttcaaattt gcaaagaagc aggagaaggc 1320

gcacttcgga atatatgcac ctctcttgag gttccaatgt ttaatccagc agaaatgctg 1380

ttcttgacag agtgggccaa cacaatgcgt ccagttgcaa aagtactcga catcttgcaa 1440

gcggaaacga atacacagct ggggtggctg ctgcctagtg tccatcagtt aagcttgaaa 1500

cttcagcgac tccaccattc tctcaggtac tgtgacccac ttgtggatgc cctacaacaa 1560

ggaatccaaa cacgattcaa gcatatgttt gaagatcctg agatcatagc agctgccatc 1620

cttctcccta aatttcggac ctcttggaca aatgatgaaa ccatcataaa acgaggcatg 1680

gactacatca gagtgcatct ggagcctttg gaccacaaga aggaattggc caacagttca 1740

tctgatgatg aagatttttt cgcttctttg aaaccgacaa cacatgaagc cagcaaagag 1800

ttggatggat atctggcctg tgtttcagac accagggagt ctctgctcac gtttcctgct 1860

atttgcagcc tctctatcaa gactaataca cctcttcccg catcggctgc ctgtgagagg 1920

cttttcagca ctgcaggatt gcttttcagc cccaaaagag ctaggcttga cactaacaat 1980

tttgagaatc agcttctact gaagttaaat ctgaggtttt acaactttga gtagcgtgta 2040

ctggcattag attgtctgtc ttatagtttg ataattaaat acaaacagtt ctaaagcagg 2100

ataaaacctt gtatgcattt catttaatgt tttttgagat taaaagctta aacaag 2156